# EUROPEAN PATENT SPECIFICATION

(11) **EP 1 466 582 B1**
(45) Date of publication and mention of the grant of the patent: **29.07.2009**
(21) Application number: 04008749.6
(22) Date of filing: 13.04.2004
(51) Int. Cl.: A61K 8/64, A61K 8/898, A61Q 5/06

(54) **Hair treatment agent comprising a silylated peptide-silane copolymer**
Haarbehandlungsmittel umfassend ein silyliertes Peptid-Silan-Copolymer
Agent de traitement capillaire comprenant un copolymère de peptide silylé-silane

(30) Priority: 11.04.2003 JP 2003107435; 14.04.2003 JP 2003108516; 15.04.2003 JP 2003109750
(43) Date of publication of application: 13.10.2004
(73) Proprietor: Seiwa Kasei Company, Limited, Higashiosaka-shi Osaka (JP)
(72) Inventor: Yoshioka, Masato c/o Seiwa Kasei Company, Ltd., Osaka (JP); Goto, Nobuyuki c/o Seiwa Kasei Company, Ltd., Osaka (JP); Uchida, Sakiko c/o Seiwa Kasei Company, Ltd., Osaka (JP); Daikai, Sueko c/o Seiwa Kasei Company, Ltd., Osaka (JP); Adachi, Takashi c/o Seiwa Kasei Company, Ltd., Osaka (JP); Tachibana, Mikuo c/o Seiwa Kasei Company, Ltd., Osaka (JP)
(74) Representative: Vossius & Partner

(56) References cited:
- EP-A- 0 934 966
- PATENT ABSTRACTS OF JAPAN vol. 2000, no. 19, 5 June 2001 (2001-06-05) & JP 2001 048757 A (SEIWA KASEI:KK), 20 February 2001 (2001-02-20)
- PATENT ABSTRACTS OF JAPAN vol. 2000, no. 05, 14 September 2000 (2000-09-14) & JP 2000 044444 A (SEIWA KASEI KK), 15 February 2000 (2000-02-15)
- PATENT ABSTRACTS OF JAPAN vol. 2000, no. 19, 5 June 2001 (2001-06-05) & JP 2001 048732 A (SEIWA KASEI:KK), 20 February 2001 (2001-02-20)
- PATENT ABSTRACTS OF JAPAN vol. 2000, no. 19, 5 June 2001 (2001-06-05) & JP 2001 048775 A (SEIWA KASEI:KK), 20 February 2001 (2001-02-20)
- PATENT ABSTRACTS OF JAPAN vol. 2002, no. 09, 4 September 2002 (2002-09-04) & JP 2002 138022 A (SEIWA KASEI:KK), 14 May 2002 (2002-05-14)
- PATENT ABSTRACTS OF JAPAN vol. 2002, no. 08, 5 August 2002 (2002-08-05) & JP 2002 121117 A (SEIWA KASEI:KK), 23 April 2002 (2002-04-23)

## Description

Present invention relates to a hair treatment agent including a hair rinse, hair conditioner, hair treatment, hair treatment cream, PPT(polypeptide) treatment, hair cream, hair spray, hair wax and the like, a shampoo and a hair coloring agent. More specifically, the present invention relates to
a hair treatment agent imparting excellent gloss and springness to hairs, making hairs smooth and improving combability of hairs, preventing formation of split hairs, and improving manageability of hairs, particularly a hair treatment agent exhibiting an excellent effect of preventing discoloration of dyed hairs, in addition to the above mentioned effects, when used for dyed hairs;
a shampoo imparting gloss to hairs, making hairs smooth and improving combability of hairs and improving manageability of hairs for the shampooed hair; and
a hair coloring agent belonging to oxidation type hair dyes, capable of dyeing hairs uniformly, giving less damage to hairs on dyeing, imparting gloss, springness and improved combability to dyed hairs, and having excellent shampoo fastness.

Conventionally used hair treatment agents such as hair rinses, hair conditioners, hair treatments and the like contain, as the conditioning agents, cationic surfactants, silicones, oily materials, hydrolyzed proteins and the like.

However, cationic surfactants soften hairs too much and deteriorate springness of hairs, although cationic surfactants exhibit good sorption action. Hydrophobic materials such as silicones and oily materials used in cosmetics are easily sorbed by little damaged hairs, that is, highly hydrophobic hairs, but hardly sorbed by damaged hairs having high hydrophilicity given by the hydrophilic groups come out to the surface of hairs caused by the damage. Consequently, the silicones and oily materials cannot exert sufficiently their properties to the damaged hairs.

Hydrolyzed proteins exhibit good sorption action, and particularly have excellent effects
of recovering deteriorated moisture-keeping actions of damaged hairs, the deterioration being caused by elution of protein components of hairs by chemical treatments such as permanent waving, dyeing or the like or by daily shampoos, and
of imparting moisture feeling to hairs. However, hydrolyzed proteins have problems that they compare unfavorably in imparting springness to hairs with high molecule silicones, and they tend to give stickiness when contained in high concentration.

Shampoos conventionally used contain as their major ingredients anionic surfactants such as alkyl sulfate, polyoxyethylenealkyl sulfate and the like, nonionic surfactants such as polyoxyethylenealkylether, fatty acid alkylol amide and the like, and ampholytic surfactants such as alkylbetaine, alkylamineoxide and the like, alone or in the mixture thereof . Furthermore, a shampoo is proposed which contains cationic surfactants to give soft and moist feeling to hairs.

However, if washing hair with the shampoo containing above described surfactants, sebum and other fat existing on the surface of hairs are excessively washed away. Consequently, the gloss and moisture of hairs are lost, the feeling of hairs is extremely deteriorated like overly dried or roughly dried hairs, and split hair and fractured hair are formed due to deterioration of combability and brushing ability.

Therefore, even in the above described shampoos, as being proposed other cosmetics for hair, an improvement of blending a silicone compound called as silicone oil and a polypeptide is proposed to exert their excellent properties together. Because the silicone oil is excellent in its extensibility, imparting gloss and luster to hair and hair protecting action resulting from imparting water-repelling property to hair, and because the polypeptide is excellent in substantivity to hair and protective and moisture-keeping actions based on film formation.

However, because silicone oils are hydrophobic (lipophilic) compounds and polypeptides are hydrophilic compounds, they are poor in compatibility with each other. Accordingly, when they are used in combination, commodity value as shampoo is apt to be impaired because the emulsion stability is low and separation is easy to occur. Also, because the polypeptides are hard to attach to a portion of the hair which has contacted the silicone oils in advance of the polypeptides, and conversely because the silicone oils are hard to attach to a portion of hair which has contacted the polypeptides in advance of the silicone oils , the combination use of the silicone oils and the polypeptides has a problem that the properties of them cannot be sufficiently exerted.

Hair coloring agents include temporally hair coloring agents, semi-permanent hair coloring agents, permanent hair coloring agents and the like, and have been widely used for beauty culture.

As the permanent hair coloring agents, an oxidation type hair coloring agent consisting of a first agent containing an oxidation dye (a dye intermediate) and a second agent containing an oxidant such as hydrogen peroxide are widely used, since various color tones are obtained and the coloring power is excellent.

However, the oxidation type hair coloring agent has problems that it takes longer time for the color reaction on hair than acidic hair coloring agents, temporally hair coloring agents containing an acid dye as the main ingredient, since the first agent containing an oxidation dye and the second agent containing an oxidant need to be mixed before applying the agent on hair, and that hardly gives uniform dyeing.

In addition, the oxidation type hair coloring agent usually contains an alkali in order to facilitate uniform penetration of an oxidation dye such that the pH is as high as 10 or more, and accordingly it has the defect that it is stimulative and the hair is easy to be damaged and the protein component in the hair is easy to be eluted. As a result there are problems that the dyed hair is overly dried, combability is deteriorated, discoloration becomes large caused by daily shampoo which elutes dye in the damaged hair, and the like.

In order to solve the problems and to improve uniform dyeability of the hair coloring agent, it is proposed to contain hydrolyzed proteins (peptide), derivatives thereof, silicones and the like in the hair coloring agent (for example, JP-A 61-55887, JP-B 3-63528, JP-B 4-4289, JP-A 5-271040).

However, the above-mentioned hydrolyzed proteins and derivatives thereof are easily washed away by shampoo after hair dyeing, and do not impart sufficiently satisfied shampoo fastness to the dyed hair, although they are sorbed to the dyed hair and impart the hair moisture feeling and gloss. Silicone is difficult to be sorbed by damaged hair in whichhydrophilic groups come out to the surface, although it is easily sorbed by undamaged hair. Further, silicone has a problem that dyeability of the hair coloring agent is lowered when the hair coloring agent contains large amount of silicone.

To solve the above described problems regarding hair treatment agent, shampoo and hair coloring agent, the inventors of the present invention have developed a silylated peptide which has silyl group added to a hydrolyzed peptide by addition reaction. And they also have tried to use it for a hair treatment agent, a shampoo and a hair coloring agent to co-exert both of the properties of hydrolyzed peptide such as imparting gloss, moisture feeling and manageability to hairs, and of the properties of silyl group such as imparting gloss to hairs and making hairs smooth (for example, JP-A 8-81338, JP-A 2002-302648, JP-A 8-81339, JP-A 8-157344).

However, because the number of silyl group is only one which is added to the peptide portion of the above described silylated peptide, there is a problem that the property imparting smoothness on the surface of hairs is inferior to high molecule silicone.

Furthermore, regarding shampoo, because the peptide portion having hydrophilicity is large, there is another problem that the amount sorbed on hairs becomes less due to being washed away in shampooing. Regarding hair coloring agent, shampoo fastness of dyed hair is not satisfactory good. Forthese reasons, the above described silylated peptide is not yet sufficiently satisfying.

Therefore, the object of the present invention is to provide a hair treatment agent in which the above-mentioned problems of the prior art are solved, which imparts good gloss and springness to hairs, improves combability of hairs, prevents formation of split hairs, improves manageability of hairs, and particularly, is excellent in a property which prevents discoloration of hairs due to shampoo, when used for dyed hairs, in addition to the properties mentioned above.

Another object of the present invention is to provide a shampoo which fully co-exerts the excellent properties of a silicone compound and a peptide, and which imparts good gloss to hairs, makes hairs smooth and improves combability of hairs, and improves manageability of hairs.

Still another object of the present invention is to provide a hair coloring agent which dyes hairs uniformly, gives little damage to hairs on dyeing, and imparts good gloss, springness, combability and the like to hairs, and makes shampoo fastness of dyed hairs excellent.

The above objects have been solved according to the present invention by the finding that a hair treatment agent, a shampoo and a hair coloring agent comprising a specific silylated peptide-silane compound copolymer composition can attain the objects.

That is, according to the present invention it has been found that a hair treatment agent prepared by comprising the specific silylated peptide-silane compound copolymer composition can attain the above object to provide properties such as imparting good gloss and springness to hairs, improving combability of hairs, preventing formation of split hairs, improving manageability of hairs, and preventing discoloration of hairs due to shampoo, when used for dyed hairs.

It also has been found that a shampoo prepared by comprising the specific silylated peptide-silane compound copolymer composition can attain the object to provide properties such as imparting good gloss to hairs, making hairs smooth and improving combability of hairs and improving manageability of hairs.

It further has been found that a hair coloring agent prepared by comprising the specific silylated peptide-silane compound copolymer composition can attain the object to provide properties such as dyeing hairs uniformly, giving little damage to hairs on dyeing, and imparting good gloss, springness, combability and the like to hairs, and making shampoo fastness of dyed hairs excellent.

The present invention provides a hair treatment agent, a shampoo or a hair coloring agent comprising a silylated peptide-silane compound copolymer composition which is obtainable by the following process and has a viscosity in the range from 500 to 20000 mPa·s in 70% of solid content concentration of said copolymer composition at 20 °C, wherein said process comprises:
polycondensing one or more kind selected from silylated peptides represented by the general formula (I) with one or more kind selected from silane compounds represented by the general formula (II) in an aqueous solution in a range of reaction molar ratio of said silylated peptide to said silane compound from 1:50 to 1:85 to form a polycondensed polymer, and then
adding by addition reaction a silane compound represented by the general formula (III) in an aqueous solution to said polycondensed polymer;
wherein said silylated peptide represented by the following general formula (I) is 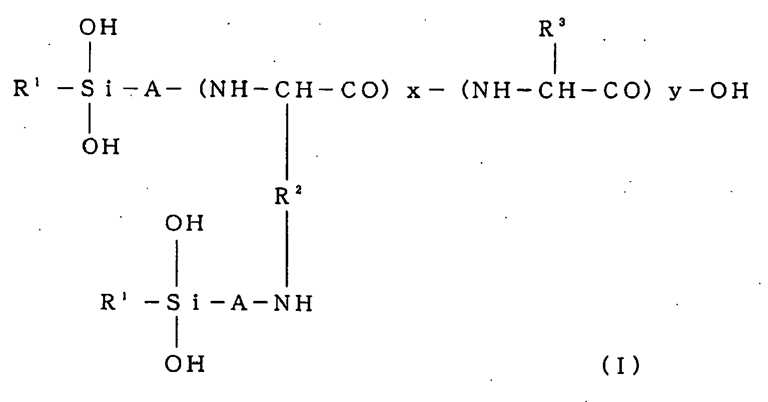 , in which R¹ represents a hydroxy group or an alkyl group having 1 to 3 carbon atoms, R² represents a residual group of a side chain obtained by removing the terminal end amino group of a basic amino acid having an amino group at the end of a side chain, R³ represents a side chain of an amino acid other than R², A is a connecting moiety and represents at least one group selected from the group consisting of -CH₂-, -(CH₂)₃-, -(CH₂)₃OCH₂CH(OH)CH₂-, -(CH₂)₃S-, -(CH₂)₃NH- and -(CH₂)₃OCOCH₂CH₂-, and x is from 0 to 50, y is from 1 to 100 and x+y is from 1 to 100, wherein x and y represent only the number of amino acid units and do not represent the order of amino acid sequence;
wherein said silane compound represented by the following general formula (II) is

R⁴mSi(OH)pY(4-p-m) (II),

in which m represents an integer from 0 to 2, p represents an integer from 2 to 4, m+p is not more than 4, R⁴ represents an organic group in which a carbon atom is directly connected to the silicon atom and the R⁴s of m may be the same or different, and the Ys of (4-p-m) represent an alkoxy group or hydrogen atom; and
wherein the silane compound represented by the following general formula (III) is

R⁵ ₃Si(OH) (III),

wherein the three R⁵s represent organic groups in which a carbon atom is directly connected to the silicon atom and the three R⁵s may be the same or different.

The letters of x and y used in the above described general formula (I) and of m, p and (4-p-m) used in the above described general formula (II) are the substitute characters.

The silylated peptide-silane compound copolymer composition used for the hair treatment agent, shampoo or hair coloring agent of the present invention may be produced by methods, for example, disclosed in JP-A 2001-48732 and in JP-A 2001-48775. Amethod forproducing said silylated peptide-silane compound copolymer composition is described in more detail hereinafter.

A silylated peptide represented above described general formula (I), which is one of the components of the silylated peptide-silane compound copolymer composition, is easily produced in an aqueous solution by methods disclosed in JP-A 8-59424 and JP-A 8-67608.

In the silylated peptide represented by the above described general formula (I), R² is a residual group of a side chain obtained by removing the terminal end amino group of a basic amino acid having an amino group at the end of a side chain. Examples of above described basic amino acid having an amino group at the end of a side chain include lysine, arginine, hydroxylysine and the like. R³ represents an amino acid side chain other than R². Examples of the amino acid include glutamic acid, aspartic acid, alanine, serine, threonine, valine, methionine, leucine, isoleucine, tyrosine, phenylalanine, proline, hydroxyproline and the like.

In the silylated peptide shown by the above described formula ( I ) , x is from 0 to 50, preferably from more than 0 to not more than 10 , y is from 1 to 100, preferably from 1 to 50, and x+y is from 1 to 100, preferably from 2 to 50. If x is more than the above range, because the number of the silyl group bonding to the amino acid in the side chains increases, the sorption action to the hair that the peptide originally possesses is decreased. If y is more than the above range, because the proportion of the silyl group portion to the peptide portion is small, the effects based on the silyl group portion cannot be sufficiently exerted. If the x+y is more than the above range, the sorbing ability and permeability to be exerted by the peptide is decreased in comparison with a low molecular weight peptide . The above described x, y and x+y are theoretically an integer. However, in case that the peptide portion is derived from a hydrolyzed peptide, since the hydrolyzed peptide is obtained in the form of a mixture of those having a different molecular weight, the measured values become average values.

The peptide portion of the silylated peptide represented by the above described formula (I) is illustrated by a natural peptide, synthetic peptide, a hydrolyzed peptide obtained by partially hydrolyzing a protein with an acid, an alkali or an enzyme, or a mixture thereof. Among them, hydrolyzed peptide is preferably used according to the reason that the protein can be conveniently obtained and the number average molecular weight of the peptide portion can be easily controlled.

Examples of the hydrolyzed peptide include animal- or vegetable-derived protein such as collagen (including gelatin of modified product thereof ) , keratin, silk fibroin (silk), sericin, casein, conchiolin, elastin, yolk protein and albumen protein of eggs such as fowl and duck, soybean protein, wheat protein, corn protein, rice (rice bran) protein and potato protein; yeast proteins separated from yeasts such as yeasts belonging to genera Saccharomyces, Candida and Endomycopsis, or yeasts such as so-called beer yeasts and sake yeasts; and peptides obtained by partially hydrolyzing microorganism-derived proteins such as proteins separated from fungi (Basidiomycetes) or Chlorella with acid, alkali or enzyme, or a mixture thereof; and the like.

A silane compound, which is another component of the silylated peptide-silane compound copolymer composition used for the hair treatment agent, shampoo or hair coloring agent of the present invention, is represented by the above described general formula (II), and said compound is obtained by hydrolyzing a silane compound represented by the following general formula (IV) in aqueous solution;

R⁶nSiX(4-n) (IV),

wherein n represents an integer from 0 to 2, R⁶ represents an organic group in which a carbon atom is directly connected to the silicon atom and the R⁶s of n may be the same or different, and the Xs of (4-n) represent at least a group selected from the group consisting of a hydroxyl group, an alkoxy group and a halogen group. The letters of n and (4-n) used in the above described general formula (IV) are the substitute characters.

Specific examples of such a silane compound represented by the general formula (IV) include tetramethoxysilane, methyltrimethoxysilane, methyldimethoxysilane, dimethyldimethoxysilane, phenyltrimethoxysilane, diphenyldimethoxysilane, hexyltrimethoxysilane, decyltrimethoxysilane, vinyltrimethoxysilane, 3-methacryloxypropyltrimethoxysilane, 3-methacryloxypropylmethyldimethoxysilane, N-(2-aminoethyl)-3-aminopropyltrimethoxysilane, N-(2-aminoethyl)-3-aminopropylmethyldimethoxysilane, 3-aminopropyltrimethoxysilane, N-phenyl-3-aminopropyltrimethoxysilane, 3-chloropropyltrimethoxysilane, 3-chloropropylmethyldimethoxysilane, 3-mercaptopropyltrimethoxysilane, 3-glycidoxypropyltrimethoxysilane, 3-glycidoxypropylmethyldimethoxysilane, dimethyloctadecyl[3-(trimethoxysilyl)propyl] ammonium chloride, 3-(trimethoxysilyl)propylpolyoxyethylene (10) ether, tetraethoxysilane, methyltriethoxysilane, methyldiethoxysilane, dimethyldiethoxysilane, phenyltriethoxysilane, diphenyldiethoxysilane, vinyltriethoxysilane, 3-methacryloxypropyltriethoxysilane, 3-methacryloxypropylmethyldiethoxysilane, N-(2-aminoethyl)-3-aminopropyltriethoxysilane, N-(2-aminoethyl)-3-aminopropylmethyldiethoxysilane, 3-aminopropyltriethoxysilane, 3-chloropropyltriethoxysilane, 3-chloropropylmethyldiethoxysilane, 3-glycidoxypropyltriethoxysilane, 3-glycidoxypropylmethyldiethoxysilane, 3-isocyanatepropyltriethoxysilane, methyldichlorosilane, methyltrichlorosilane, dimethyldichlorosilane, phenyltrichlorosilane, diphenyldichlorosilane, vinyltrichlorosilane, 3-chloropropylmethyldichlorosilane and the like.

The reaction of the silylated peptides represented by the above described general formula (I) with the silane compound represented by the general formula (II) is carried out according to, for example, the following procedure:
Preparing an aqueous solution of the silylated peptides represented by the above described general formula (I) to become acidic with hydrochloric acid, sulfuric acid or the like, or to become basic with an aqueous sodium hydroxide solution, an aqueous potassium hydroxide solution or the like;
Adding dropwisely the silane compound represented by the general formula (IV) to said solution to form a silane compound represented by the general formula (II) having at least two hydroxyl groups directly connected to a silicon atom by hydrolyzing an alkoxy group, a halogen group and the like of the silane compound represented by the above described general formula (IV);
Neutralizing the solution obtained thereby to polycondense a hydroxyl group of the organic silane compound having a hydrophilic group represented by the general formula (I) with a hydroxyl group of the silane compound represented by the general formula (II).
Thus, a silylated peptide-silane compound copolymer composition is obtained.

The hydrolysis reaction generally proceeds in a state of pH 1 to 3. However, because certain kinds of silylated peptide represented by the general formula (I) are likely to form insoluble materials in acidic state, when such silylated peptide is used, the hydrolysis is preferably conducted in the state of pH 10 to 11. When an alkoxysilane compound is used as the silane compound represented by the general formula (IV), pH control is required only before adding dropwisely the alkoxysilane compound. However, when the reaction is conducted in basic state by using a halogenated silane compound or carboxysilane compound as the silane compound represented by the general formula (IV) , it is necessary to keep the pH between 10 and 11 by adding an aqueous sodium hydroxide solution, an aqueous potassium hydroxide solution or the like due to pH of the reaction solution decreasing during the reaction. Further, when the reaction is conducted in acidic state by using an amino silane compound as the silane compound represented by the general formula (IV), it is necessary to keepthe pH between 1 and 3 by adding diluted hydrochloric acid, diluted sulfuric acid or the like due to pH of the reaction solution increasing during the reaction.

The reaction temperature is preferably from 30 to 60 °C, because the reaction does not proceed smoothly when the reaction temperature is too low, and because an alkoxy group or a halogen group of the silane compound represented by the above described general formula (IV) is hydrolyzed steeply when the reaction temperature is too high. In terms of the reaction time, though it varies depending on the amount of the reaction solution, it is preferable to add dropwisely the silane compound represented by the above described general formula (IV) to said solution for from 2 to 6 hours, and then the solution is kept stirred for from 5 to 20 hours after said addition is completed.

At completion of the hydrolysis reaction, because the reaction solution is in acidic or basic state, the solution is neutralized by adding an aqueous alkali solution such as an aqueous sodium hydroxide solution, aqueous potassium hydroxide solution and the like when the reaction solution is acidic, or by adding an acid aqueous solution such as dilute hydrochloric acid, dilute sulfuric acid and the like when the reaction solution is basic; and then the solution is stirred to be neutralized. By this neutralization, polycondensation is further proceeded to give a silylated peptide-silane compound copolymer composition. Stirring time conducted after the neutralization is preferably for from 1 to 10 hours.

A silylated peptide-silane compound copolymer composition having better compatibility in a hair treatment agent containing a large amount of oily material can be produced by suitably selecting the kind and reaction amount of the silane compound represented by the general formula (II) in the polycondensation of the silylated peptide represented by the general formula (I) with the silane compound represented by the general formula (II).

That is, the silylated peptide-silane compound copolymer composition has surface activity, since it is produced by polycondensation of a hydrophilic peptide portion in the silylated peptide and the silane compound havig hydrophobic group. As the result, when the hair treatment agent contains a higher fatty acid and a higher alcohol having long carbon chain, stability of the higher fatty acid and the higher alcohol having long carbon chain can be improved, if a silane compound represented by the general formula (IV) and connecting an organic group as R⁶ having a longer carbon chain, such as hexyltrimethoxysilane, decyltrimethoxysilane and the like is used for polycondensing with the silylated peptide.

Thus, by selecting the kind of the silane compound to be reacted, a silanecompound copolymer composition having various properties can be obtained.

The silylated peptide-silane compound copolymer composition used for a hair treatment agent, a shampoo or a hair coloring agent of the present invention is produced by polycondensing one or more kind selected from silylated peptide represented by the above described general formula (I) with one or more kind selected from silane compound represented by the above described general formula (II) in a range of reaction molar ratio of from 1:50 to 1:85.

When the reaction molar ratio of the silylated peptide representedby the general formula (I) to the silane compound represented by the general formula (II) is less than the above described range, a hair treatment agent containing said copolymer composition is impossible to provide properties such as imparting gloss and springness to hairs; a shampoo containing said copolymer composition is impossible to provide properties such as imparting gloss to hairs, making hairs smooth and improving combability of hairs; and
a hair coloring agent containing said copolymer composition is impossible to provide properties such as giving little damage to hairs on dyeing, imparting good gloss and springness to hairs, and exhibiting good shampoo fastness.

When the reaction molar ratio of the silylated peptide represented by the general formula (I) to the silane compound represented by the general formula (II) is more than the above described range, the hair treatment agent, the shampoo and the hair coloring agent not only is unable to achieve the increase of properties imparting gloss, springness and smoothness to meet the value of the reaction molar ratio but causes silicon-oil touch and further may deteriorate the handling ability because of high viscosity of the silylated peptide-silane compound copolymer composition.

Because a hydroxyl group remains at terminal silylate group of the silylated peptide-silane compound copolymer composition obtained by above described procedure, the silylated peptide-silane compound copolymer compositions may coagulate themselves and, as a result, be highly polymerized. Therefore, a silane compound represented by the above described general formula (III) which produces with hydrolysis one hydroxyl group directly connecting to the silicon atom is reacted with said copolymer composition.

The silane compound represented by the above described general formula (III) is obtained by hydrolyzing a silane compound represented by the following general formula (V) in aqueous solution

R⁷ ₃SiZ (V)

wherein the three R⁷s represent organic groups in which a carbon atom is directly connected to the silicon atom and the three R⁷s may be the same or different, Z is at least one group selected from the group consisting of a hydroxyl group, an alkoxy group and a halogen group.

Examples of the silane compound represented by the general formula (V) which produces with hydrolysis one hydroxyl group directly connecting to the silicon atom, include dimethylvinylchlorosilane, n-butyldimethylchlorosilane, tert-butyldimethylchlorosilane, tert-butyldiphenylchlorosilane, octadecyldimethylchlorosilane, methyldiphenylchlorosilane, tri-n-butylchlorosilane, triethylchlorosilane, trimethylchlorosilane, tri-n-propylchlorosilane, triphenylchlorosilane, trimethyliodidesilane, trimethylethoxysilane, dimethylvinylethoxysilane, dimethylvinylmethoxysilane, trimethylethoxysilane, trimethylmethoxysilane, triphenylethoxysilane and the like.

In addition, silyl compounds having two silicon atoms such as hexamethyldisilazane and hexamethyldisiloxane can also be used because these compounds generate one hydroxyl group directly connecting to a silicon atom with hydrolysis .

Since a silane compound represented by the general formula (V) which produces by hydrolysis one hydroxyl group directly connecting to silicon atom has only one reactive group directly connected to a silicon atom; a silane compound represented by the general formula (III) obtained by hydrolyzing the above said silane compound reacts with a hydroxyl group existing in the silylated peptide-silane compound copolymer composition; and reduces the hydroxyl group in said silylated peptide-silane compound copolymer composition; and thus prevents said silylated peptide - silane compound copolymercompositionfrom further polycondensing by themselves. Namely, by reacting the silane compound represented by the general formula (III), a silylated peptide-silane compound copolymer composition having excellent storage stability can be obtained.

A reaction of a silylated peptide-silane compound copolymer composition with a silane compound represented by the general formula (III) is conducted, for example, by adding dropwisely the silane compound represented by the general formula (V) into an aqueous solution of the silylated peptide-silane compound copolymer composition; and thus a hydroxyl group of the silylated peptide-silane compound copolymer composition is condensed with a hydroxyl group of the silane compound represented by the general formula (III).

In case using a silane compound, in which Z represents a halogen atom, as the silane compound represented by the above described general formula (V), because said silane compound shows excellent hydrolysis property, the above-described reaction can be conducted by directly adding dropwisely the silane compound represented by the above described general formula (V) into an aqueous solution of the silylated peptide-silane compound copolymer composition. However, in case using a silane compound, in which Z represents an alkoxy group or a silane compound containing two silicon atoms such as hexamethyldisiloxane, as the silane compound represented by the above described general formula (V), it is necessary that said silane compound is previously hydrolyzed in an aqueous solution having a pH 2 to 3 to give a silane compound represented by the general formula (III) and then the silane compound obtained by hydrolysis is added dropwisely into an aqueous solution of the silylated peptide-silane compound copolymer composition.

The reaction temperature of an silylated peptide-silane compound copolymer composition with a silane compound represented by the above described general formula (V) is preferably from 30 to 60 °C. The reaction time, though it varies depending on the amount of reaction solution, is preferably from 30 minutes to 2 hours to add dropwisely the silane compound represented by the general formula (V) , and then from 1 to 6 hours to stir the reaction solution after said addition is completed.

After completion of the stirring, the reaction solution is neutralized with an aqueous alkali solution such as an aqueous sodium hydroxide solution, an aqueous potassium hydroxide solution and the like; and the reaction is completed by further stirring for 1 to 10 hours to obtain a silylatedpeptide-silane compound copolymer composition. After controlling the pH and the concentration of the reaction solution, the silylated peptide-silane compound copolymer composition having a viscosity in a range from 500 to 20000 mPa· s in 70% of solid content concentration of said copolymer composition at 20 °C is used for a hair treatment agent, a shampoo or a hair coloring agent of the present invention.

The reason why an amount of the silylated peptide-silane compound copolymer composition contained in the hair treatment agent, the shampoo or the hair coloring agent of the present invention is set in the range in terms of the viscosity ranging from 500 to 20000 mPa·s in 70% of solid content concentration of said copolymer composition at 20 °C is; that the effect of the silylated peptide-silane compound copolymer composition may not be realized due to insufficient polymerization when the viscosity in 70% of solid content concentration of said copolymer composition at 20 °C is less than the above describedrange; andthat the handling ability of the silylated peptide-silane compound copolymer composition is deteriorated due to the lack of fluidity when the viscosity is more than the above described range.

The hair treatment agent of the present invention includes a hair rinse, hair conditioner, hair treatment, hair treatment cream, PPT(polypeptide) treatment, hair cream, hair spray, hair wax and the like, a shampoo, a hair coloring agent, and the like, and comprises the above-mentioned a silylated peptide-silane compound copolymer composition.

In general, the amount contained in the hair treatment agent of the present invention (that is, the amount blended to the hair treatment agent) of the silylated peptide-silane compound copolymer composition is preferably from 0.01 to 5% by weight in the hair treatment agent. However, the preferable amount varies depending on the kind and method of use of the hair treatment agent, since it may cause roughness and stickiness on the hair due to the water-insoluble property of the silylated peptide-silane compound copolymer composition and to accumulation of said copolymer composition in daily use when the amount contained of the silylated peptide-silane compound copolymer composition is too large.

Therefore, the preferable amount is 0.01 to 1% by weight in the hair treatment agent for a hair rinse to be used daily; and the preferable amount is 0 . 05 to 5% by weight for a hair treatment applied to damaged hairs due to a chemical treatment such as a permanent wave treatment or dyeing hair. When a dyed hair treatment to be used after dyeing hairs contains the silylated peptide-silane compound copolymer composition in an amount of 0.05 to 5% by weight, elimination of dye from the dyed hair is suppressed. Regarding any kinds of hair treatment agent, the effects of imparting excellent gloss and springness to hairs, improving combability of hairs, preventing formation of split hairs, improving manageability of hairs, and preventing discoloration of dyed hairs due to shampoo, when used for dyed hairs, may not be attained, when the content of the silylated peptide-silane compound copolymer composition is 0.01% by weight or less.

Of a shampoo of the present invention, the amount contained in the shampoo (that is, the amount blended to the shampoo) of the silylated peptide-silane compound copolymer composition having a viscosity in the range from 500 to 20000 mPa·s in 70% of solid content concentration of said copolymer composition at 20 °C is preferably from 0.01 to 5% by weight, more preferably from 0 . 05 to 2 . 5% by weight. It may be unable to fully obtain the effects imparting gloss to hairs, making hairs smooth, improving combability and manageability of hairs, when the amount contained of the silylated peptide-silane compound copolymer composition is less than the above described range. It may cause roughness and stickiness on the shampooed hair due to the water-insoluble property of the silylated peptide-silane compound copolymer composition and to accumulation of said copolymer composition in daily use when the amount contained of the silylated peptide-silane compound copolymer composition is more than the above described range. In blending to the shampoo, the silylated peptide-silane compound copolymer composition may be used alone or in a mixture of two kinds or of more than two kinds.

The shampoo of the present invention may be produced in a conventional manner except for containing the silylated peptide-silane compound copolymer composition having a viscosity in a range from 500 to 20000 mPa·s in 70% of solid content concentration of said copolymer composition at 20 °C.

For example, various surfactants used conventionally may be used for a surfactant used as a major ingredient of the shampoo. For example, anionic surfactants, nonionic surfactants, ampholytic surfactants, cationic surfactants, and the like may be used alone or in a mixture of two or more than two kinds. The amount of the surfactant used is usually from 0.1 to 25% by weight in the shampoo, more preferably from 2 to 25% by weight, and further more preferably from 5 to 15% by weight.

Examples of the anionic surfactants include alkyl sulfates such as ammonium lauryl sulfate, lauryl ethanolamine sulfate, sodium lauryl sulfate, lauryl triethanolamine sulfate and the like; polyoxyethylene alkyl ether sulfates such as polyoxyethylene(2EO)laurylether triethanolamine sulfate (,wherein EO represents ethylene oxide and the value attached on the head of letter of EO represents the number of ethylene oxide molecules added), sodium polyoxyethylene(3EO)alkyl(,wherein said alkyl group is selected from the group having carbon atoms from 11 to 15 or from a mixture of more than two kinds thereof, and is abbreviated in a manner such as "C11-15" hereinafter)ether sulfate, and the like; alkylbenzene sulfates such as sodium laurylbenzene sulfate, laurylbenzene triethanolamine sulfate and the like; polyoxyethylene alkyl ether acetates such as sodium polyoxyethylene(3EO)tridecylether acetate and the like; sodium alkane sulfate, sodium hydrogenated(palm oil fatty acid)monoglyceride sulfate, disodium undecylenoylamideethyl sulfosuccinate, sodium octylphenoxydiethoxyethyl sulfate, disodium oleamide sulfosuccinate, sodium dioctyl sulfosuccinate, disodium laurylsulfosuccinate,polyoxyethylenealkyl(C12-16)ether phosphate(2-12 EO), sodium polyoxyethyleneoleylether phosphate, sodium polyoxyethylenecetylether phosphate, disodium polyoxyethylenelaurylether sulfosuccinate, sodium polyoxyethylenelaurylether phosphate, sodium lauryl sulfosuccinate, sodium tetradecene sulfonate and the like.

Examples of nonionic surfactants include polyoxyethylenealkyl(C12-14) ether(7EO), polyoxyethyleneoctylphenyl ether, polyoxyethyleneoleyl ether, glyceryl polyoxyethylene oleate, polyoxyethylenestearyl ether, polyoxyethylenecetyl ether, polyoxyethylenecetylstearyl diether, polyoxyethylenesorbitol lnolin acid(40EO), polyoxyethylenenonylphenyl ether, polyoxyethylenepolyoxypropylenecetyl ether, polyoxyethylenepolyoxypropylenedecyltetradecyl ether, decylpolyglycoxyde, laurylpolyglycoxyde, cetylpolyglycoxyde, polyoxyethylenelanolin alcohol, polyoxypropylenestearyl ether and the like.

Examples of ampholytic surfactants include 2-alkyl-N-carboxylmethyl-N-hydroxyethyl imidazolinium betaine, undecylhydroxyethyl imidazolinium betaine, undecyl-N-hydroxyethyl-N-carboxylmethyl imidazolinium betaine, stearyldihydroxyethyl betaine, stearyldiethyl betaine, cocoalkyldimethyl glycine, cocoamidopropyldimethyl glycine, sodium cocoalkyl-N-carboxyethyl-N-hydroxyethyl imidazolinium betaine, disodium cocoalkyl-N-carboxyethoxyethyl-N-carboxyethyl imidazolinium hydroxide, DL-pyrrolidonecarboxylic acid salt of L-cocoylarginine ethyl ester and the like.

Examples of cationic surfactants include stearyltrimethyl ammonium chloride, cetyltrimethyl ammonium chloride, cetostearyltrimethyl ammonium chloride, stearylbis(diethyleneglycol)hydroxyethyl ammonium chloride, behenyltrimethyl ammonium chloride, distearyldimethyl ammonium bromide, stearyldimethylbenzyl ammonium chloride, behenyltrimethyl ammonium bromide, cetyltrimethyl ammonium iodide, oleylbenzyldimethyl ammonium chloride, oleylbis[polyoxyethylene(15EO)]methyl ammonium chloride, tri[polyoxyethylene(5EO)]stearyl ammonium chloride, alkyltrimethyl ammonium chloride, polyoxypropylenemethyldiethyl ammonium chloride, mink oil amidepropyldimethylhydroxyethyl ammonium chloride, alkylpyridinium salt, γ-gluconamidepropyldimethylhydroxyethyl ammonium chloride, and the like.

The amount contained in the hair coloring agent (that is, the amount blended to the hair coloring agent) of the silylated peptide-silane compound copolymer composition is preferably from 0.05 to 10% by weight, particularly preferably from 0.5 to 5% by weight. It may be unable to fully obtain the effects suppressing damage of hairs on dyeing and imparting gloss, springness and good combability to hairs, and shampoo fastness of dyed hairs, when the amount contained of the silylated peptide-silane compound copolymer composition is less than the above described range. Improvement of the effects meeting the increase of the amount scarcely realizes, and it may cause stickiness on the hair and oily feeling due to a large amount of the silylated peptide-silane compound copolymer composition remained on the hair, when the amount contained of the silylated peptide-silane compound copolymer composition is more than the above described range.

For a two-package type hair coloring agent, one embodiment of the present invention, the silylated peptide-silane compound copolymer composition may be comprised either in a first composition or in a second composition. In general, it is more suitable to be comprised in the first composition. In general, the first composition of a two-package type hair coloring agent comprises an oxidation dye (dye intermediate) and an alkali agent, and further, based on demand, a nitro dye.

Examples of the oxidation dye include a phenylenediamine compound such as p-phenylenediamine, N-phenyl-p-phenylenediamine, 4,4'-diaminodiphenylamine or o-phenylenediamine, a tolueneamine compound such as a toluene-2,5-diamine or toluene-3,4-diamine, an aminophenol compound such as p-aminophenol, p-methylaminophenol or o-methylaminophenol, an aminonitrophenol such as o-amino-m-nitrophenol, a diaminopyridine such as 2,6-diaminopyridine, and the like. Generally used couplers include, for example, m-phenylenediamine, toluene-2,4-diamine, m-aminophenol, naphthol, resorcinol, catechol, hydroquinone and the like.

The alkali agent is not particularly limited, and examples thereof include sodium hydroxide, potassium hydroxide, aqueous ammonia, monoethanol amine, triethanol amine, and the like. Examples of nitro dye include 2-amino-4-nitrophenol, nitro-p-phenylenediamine, p-nitro-o-phenylenediamine, 2-amino-5-nitrophenol, and the like.

The second composition contains an oxidizing agent as the essential component, and the oxidizing agent is not particularly limited. Examples of the oxidizing agent used in the second composition include hydrogen peroxide, sodium percarbonate, sodium perborate, sodium peroxide, and the like.

Hair coloring agent of the present invention comprises a silylated peptide-silane compound copolymer composition having a viscosity in the range from 500 to 20000 mPa·s in 70% of solid content concentration of said copolymer composition at 20 °C, as the essential component, and also comprises another component as long as it is usable in the hair coloring agent. Preparation of the hair coloring agent of the present invention can be conducted either by adding a silylated peptide-silane compound copolymer composition havinga viscosity in the range from 500 to 20000 mPa·s in 70% of solid content concentration of said copolymer composition at 20 °C to an already prepared hair coloring agent, or by adding a silylated peptide-silane compound copolymer composition havinga viscosity in the range from 500 to 20000 mPa·s in 70% of solid content concentration of said copolymer composition at 20 °C together with other component simultaneously.

As mentioned above, a hair treatment agent of the present invention essentially can be prepared by adding a silylated peptide-silane compound copolymer composition having a viscosity in the range from 500 to 20000 mPa·s in 70% of solid content concentration of said copolymer composition at 20 °C to a conventional hair treatment agent. Furthermore, other suitable components may be added to the hair treatment agent of the present invention in the range not to deteriorate the effects of the present invention.

Example of the other suitable component include anionic surfactants, nonionic surfactants, cationic surfactants, ampholytic surfactants, cationic polymers, ampholytic polymers, anionic polymers, thickener, extracts from animals and vegetables, polysaccharide or derivatives thereof, hydrolyzed peptides of proteins derived from animals, vegetables and microorganism or derivatives thereof, wetting agent, lower alcohols, higher alcohols, amino acids, fats and oils, silicones, preservatives, perfumes, and the like.

A shampoo of the present invention essentially comprises a silylated peptide-silane compound copolymer composition having a viscosity in the range from 500 to 20000 mPa·s in 70% of solid content concentration of said copolymer composition at 20 °C and a surfactant, and said shampoo is produced by dissolving said those essential components into water or a solution comprising water and a suitable solvent. Furthermore, other suitable components may be added in the range not to deteriorate the effects of the present invention.

Examples of said other suitable components are illustrated as follows:
A synthetic polymer such as a cationic polymer, ampholytic polymer and anionic polymer including cationized cellulose, cationized guar gum, polydiallyldimethyl ammonium chrolide, polyvinylpyrrolidone, polyethyleneimine and the like; A thickners such as isostearic acid diethanolamide, undecylicacid monoethanolamide,oleicacid diethanolamide, monoethanolamine tallow acid amide, hydrogenated tallow acid diethanolamide, stearic acid diethanolamide, stearic acid diethylaminoethylamide, stearic acid monoethanolamide, myristic acid diethanolamide, coconut fatty acid ethanolamide, coconut fatty acid diethanolamide, lauric acid isopropanolamide, lauric acid ethanolamide, lauric acid diethanolamide, lanoline fatty acid diethanolamide, and the like; a fat and an oil such as waxes, paraffin, fatty acid esters, glyceride, oils derived from animals and vegetables, and the like; a moisturizing agent such as a substance extracted from animals and vegetables, polysaccharide or its derivatives, propyleneglycol, 1,3-butyleneglycol, ethyleneglycol, glycerin, and the like; a lower alcohol such as ethanol, propanol, isopropanol, and the like; a higher alcohol such as cetanol, stearyl alcohol, oleyl alcohol, behenyl alcohol, and the like; an amino acid such as L-aspartic acid, sodium L-aspartate, DL-alanine, L-arginine, glycine, L-glutamic acid, L-cystine, L-threonine, and the like; a licorice derivative such as glycyrrhizinic acid, disodium carbenoxolone, and the like; an antiphlogistic such as allantoin, guaiazulene, aloe, a-bisabolol, and the like; an anti-dandruff agent such as salicylic acid, zinc pyrithione, pyroctone olamine, sulfur, selenium sulfide, triclosan, resorcin, vitamin A, vitamin B₆, and the like; a hydrolyzed protein derived from animal, vegetable and microorganism such as soy bean , wheat, corn, potato, yeast, fungi and the like, and a derivative thereof such as N-quaternary ammonium derivatives, N-acylated derivatives, N-silylated derivatives, esterified derivatives, and the like, wherein said animal-, vegetable- and microorganism-derived protein include collagen, keratin, silk, serine, casein, and the like; a silicon derivative such as chained or cyclic metylpolysiloxane, methylphenylpolysiloxane, dimethylpolysiloxane-polyethyleneglycolcopolymer, amino modified silicon, quaternary ammonium modified silicon, and the like.

A hair coloring agent of the present invention may further comprise other suitable components in the range not to deteriorate the effects of the present invention, as conventional hair coloring agents, in addition to the above-mentioned essential components.

Example of the other suitable component include surfactants such as nonionic surfactants, anionic surfactants, cationic surfactants, ampholytic surfactants, a dissolving assistant such as glycerol or propylene glycol, a humectant, a viscosity modifier such as carboxymethylcellulose, hydroxyethylcellulose, carboxy vinylpolymer or polyacryl amide, copolymer of acrylates, methacrylates or the like, polymers such as N,N-dimethylaminoethylmethacrylate copolymer, copolymer of vinylpyrrolidone and vinyl acetate or the like, pH adjusting agents, perfumes, presevatives, antioxidants, chelating agents, and the like.

The hair treatment agent of the present invention imparts good gloss and springness to hairs, improves combability of hairs, prevents formation of split hairs, improves manageability of hairs, and particularly, is excellent in a property which prevents discoloration of hairs due to shampoo, when used for dyed hairs, due to the silylated peptide-silane compound copolymer composition having a viscosity in the range from 500 to 20000 mPa·s in 70% of solid content concentration of said copolymer composition at 20 °C contained in the composition.

The shampoo of the present invention imparts gloss to hairs, makes hairs smooth, improves combability and manageability of hairs.

The hair coloring agent of the present invention dyes hairs uniformly, gives little damage to hairs on dyeing, and imparts good gloss, springness, combability and the like to hairs, and makes shampoo fastness of dyed hairs excellent.

### [Examples]

The present invention is more specifically described and explained by means of the following Examples, but is not limited the scope thereof. In the following Example and Comparative Example, the amount of each component blended is represented by the part by weight; and if the amount blended is not the amount of solid content of said component, the concentration of said solid content is represented by being enclosed in parentheses followed to said component name. The percent (%) showing concentration is % by weight. In some Examples and Comparative Examples, a term "blend" is used in stead of "contain". Prior to the Example, examples producing a silylated peptide-silane compound copolymer composition used in the Example are described as Production Example.

### Production Example 1

### Production of N-[2-hydroxy-3-(3'-methyldihydroxysilyl)propoxy]propyl hydrolyzed sericin-dimethyldiethoxysilane-octyltriethoxysilane copolymer composition [1:25:25 (molar ratio)]

Into the reaction vessel made of glass shaped in cylinder with round bottom of its inner diameter of 12 cm and its volume of 2 liters, 200 g of 10% aqueous solution of N-[2-hydroxy-3-(3'-methyldihydroxysilyl)propoxy]propyl hydrolyzed sericin ( , wherein the number average molecular weight of said hydrolyzed sericin is about 500) and 11.5 g of 18% aqueous solution of hydrochloric acid were added to adjust pH of a mixture to 1.5 and said mixture was heated up to 60 °C. While the mixture being stirred in 400 rpm, the mixture of 99.1 g of dimethyldiethoxysilane (a trade name of KBE-22, manufactured by Shin-Etsu Silicone Co., Ltd.) and 184.7 g of octyltriethoxysilane (a trade name of A-137, Nippon Unicar Co., Ltd.) was dropped into said mixture for 5 hours, and then, after said dropping being completed, the solution obtained was further stirred for 15 hours at 60 °C. Then, while the solution being stirred, 40.8 g of 5% aqueous solution of sodium hydroxide was gradually dropped into said solution to adjust the solution pH to 6, and the solution was further stirred for one hour at 60 °C. While the reaction solution obtained being stirred in 400 rpm at 60°C, 11.6 g of trimethylchlorosilane (a trade name of KA-31, manufactured by Shin-Etsu Silicone Co. , Ltd. ) was added, and then said reaction solution was stirred for one hour at 60 °C. Then, after 78.8 g of 5% aqueous solution of sodium hydroxide being dropped into the reaction solution and pH of said solution being adjusted to 6, the solution was stirred for one hour at 60 °C, and then the solution was heated up to 80 °C and further stirred for one hour. Then, the reaction solution was concentrated to the solid content of 70% by vacuum concentration with the rotary-evaporator.
Thus, 260 g of N-[2-hydroxy-3-(3'-methyldihydroxysilyl)propoxy]propyl hydrolyzed sericin-dimethyldiethoxysilane-octyltriethoxysilane copolymer composition was obtained.

The viscosity of 70% aqueous solution at 20 °C of obtained N-[2-hydroxy-3-(3'-methyldihydroxysilyl)propoxy]propyl hydrolyzed sericin-dimethyldiethoxysilane-octyltriethoxysilane copolymer composition was measured by B-type viscometer with the rotor 3 and number of revolution 30, thus the viscosity resulted in 2120 mPa·s.

### Production Example 2

### Production of N-[2-hydroxy-3-(3'-methyldihydroxysilyl)propoxy]propyl hydrolyzed collagen-dimethyldiethoxysilane-octyltriethoxysilane copolymer composition [1:25:25 (molar ratio)]

Into the reaction vessel made of glass shaped in cylinder with round bottom of its inner diameter of 12 cm and its volume of 2 liters, 150 g of 10% aqueous solution of N-[2-hydroxy-3-(3'-methyldihydroxysilyl)propoxy]propyl hydrolyzed collagen ( , wherein the number average molecular weight of hydrolyzed collagen is about 500) and 7.6 g of 18% aqueous solution of hydrochloric acid were added to adjust pH of the mixture to 1.5 and said mixture was heated up to 60 °C. While the mixture being stirred in 400 rpm, the mixture of 79.7 g of dimethyldiethoxysilane (a trade name of KBE-22, manufactured by Shin-Etsu Silicone Co., Ltd.) and 148.6 g of octyltriethoxysilane (a trade name of A-137, Nippon Unicar Co., Ltd.) was dropped into said mixture for 5 hours, and then, after said dropping being completed, the solution obtained was further stirred for 15 hours at 60 °C. Then, while the solution being stirred, 22.9 g of 5% aqueous solution of sodium hydroxide was gradually dropped into said solution to adjust the solution pH to 6, and the solution was further stirred for one hour at 60 °C. While the reaction solution obtained being stirred in 400 rpm at 60 °C, 9 . 3 g of trimethylchlorosilane (a trade name of KA-31, manufactured by Shin-Etsu Silicon Co. , Ltd.) was added, and then said reaction solution was stirred for one hour at 60 °C. Then, after 68.5 g of 5% aqueous solution of sodium hydroxide being dropped into the reaction solution and pH of said solution being adjusted to 6, the solution was stirred for one hour at 60 °C, and then the solution was heated up to 80 °C and further stirred for one hour. Then, the reaction solution was concentrated to the solid content of 70% by vacuum concentration with the rotary-evaporator.
Thus, 211.9 g of N-[2-hydroxy-3-(3'-methyldihydroxysilyl)propoxy]propyl hydrolyzed collagen-dimethyldiethoxysilane-octyltriethoxysilane copolymer composition was obtained.

The viscosity of 70% aqueous solution at 20 °C of obtained N-[2-hydroxy-3-(3'-methyldihydroxysilyl)propoxy]propyl hydrolyzed collagen-dimethyldiethoxysilane-octyltriethoxysilane copolymer composition was measured according to the same condition applied in Production Example 1, thus the viscosity resulted in 1116 mPa·s.

### Production Example 3

### Production of N-[2-hydroxy-3-(3'-methyldihydroxysilyl)propoxy]propyl hydrolyzed silk-dimethyldiethoxysilane-octyltriethoxysilane copolymer composition [1:40:40 (molar ratio)]

Into the reaction vessel made of glass shaped in cylinder with round bottom of its inner diameter of 12 cm and its volume of 2 liters, 127.3 g of 10% aqueous solution of N-[2-hydroxy-3-(3'-methyldihydroxysilyl)propoxy]propyl hydrolyzed silk (, wherein the number average molecular weight of hydrolyzed silk is about 600) and 4.8 g of 18% aqueous solution of hydrochloric acid were added to adjust pH of the mixture to 1.5 and said mixture was heated up to 60 °C. While the mixture being stirred in 400 rpm, the mixture of 88.0 g of dimethyldiethoxysilane (a trade name of KBE-22, manufactured by Shin-Etsu Silicon Co., Ltd.) and 164.0 g of octyltriethoxysilane (a trade name of A-137, Nippon Unicar Co. , Ltd. ) was dropped into said mixture for 5 and half hours, and then, after said dropping being completed, the solution obtained was further stirred for 15 hours at 60 °C . Then, while the solution being stirred, 17.1 g of 5% aqueous solution of sodium hydroxide was gradually dropped into said solution to adjust the solution pH to 6, and the solution was further stirred for one hour at 60 °C. While the reaction solution obtained being stirred in 400 rpm at 60 °C, 6 . 4 g of trimethylchlorosilane (a trade name of KA-31, manufactured by Shin-Etsu Silicon Co. , Ltd. ) was added, and then said reaction solution was stirred for one hour at 60 °C. Then, after 45. 7 g of 5% aqueous solution of sodium hydroxide being dropped into the reaction solution and pH of said solution being adjusted to 6, the solution was stirred for one hour at 60 °C, and then the solution was heated up to 80 °C and further stirred for one hour. Then, the reaction solution was concentrated to the solid content of 70% by vacuum concentration with the rotary-evaporator.
Thus, 209 g of N-[2-hydroxy-3-(3'-methyldihydroxysilyl)propoxy]propyl hydrolyzed silk-dimethyldiethoxysilane-octyltriethoxysilane copolymer composition was obtained.

The viscosity of 70% aqueous solution at 20 °C of obtained N-[2-hydroxy-3-(3'-methyldihydroxysilyl)propoxy]propyl hydrolyzed silk-dimethyldiethoxysilane-octyltriethoxysilane copolymer composition was measured by the same condition applied in Production Example 1, thus the viscosity resulted in 640 mPa·s.

### Example 1 and Comparative Examples 1 and 2

Three kinds of hair treatment agents shown in Table 1 were prepared. After applying the hair treatment agents on dyed hairs respectively, gloss, springness, combability and manageability of the treated hairs were evaluated. In addition, degree of discoloration of the treated hairs by shampooing was also visually compared with naked eyes.

In Example 1, N-[2-hydroxy-3-(3'-methyldihydroxysilyl)propoxy]propyl hydrolyzed sericin-dimethyldiethoxysilane-octyltriethoxysilane copolymer composition produced in Production Example 1, using a silylated peptide-silane compound copolymer composition, was used. In Comparative Example 1, in place of a silylated peptide-silane compound copolymer composition, polyoxyethylene/methylpolysiloxane copolymer, a polyether modified silicone, was used; in Comparative Example 2, neither a silylated peptide-silane compound copolymer composition nor a silicone were used. The numbers representing amounts in all Tables in Examples and Comparative examples in this specification are weight parts unless otherwise stated.

**Table 1**

| | Example | Comparative example | |
|---|---|---|---|
| | 1 | 1 | 2 |
| Silylated peptide-silane compound copolymer composition (70%) of Production Example 1 | 3.0 | 0 | 0 |
| Polyoxyethylene/methylpoly siloxane copolymer*1 | 0 | 3.0 | 0 |
| Sodium acrylate/acryloyldimethyl Taurine copolymer +isohexadecane+ Polysorbate80 mixture *2 | 2.0 | 2.0 | 2.0 |
| 1,3-butleneglycol | 2.0 | 2.0 | 2.0 |
| glycerol | 1.0 | 1.0 | 1.0 |
| Liquid paraffin#70 | 1.0 | 1.0 | 1.0 |
| Squalane | 2.0 | 2.0 | 2.0 |
| Hydrolyzed silk (7%) *3 | 0.5 | 0.5 | 0.5 |
| Paraoxybenzoate+phenoxy ethanol mixture *4 | 0.5 | 0.5 | 0.5 |
| Sterilized ion-exchange water | Volume adjustable totally 100 part | Volume adjustable totally 100 part | Volume adjustable totally 100 part |

| | | | |
|---|---|---|---|
| *1 : SH3775M (trade name) , manufactured by Dow Corning Toray Silicone Co.,Ltd. *2: Simul gel EG (trade name), manufactured by Seppic Co.,Ltd. *3: Promois silk-1000 ( trade name), manufactured by SEIWA KASEI Co., Ltd. *4: Seisept H(trade name, manufactured by SEIWA KASEI Co. , Ltd. | | | |

Dyed hair samples to be treated for evaluation were prepared by washing, bleaching and dyeing hairs.

More concrete steps are as follows:
Preparing three bundles of hair, each bundle being the length of 13 cm and the weight of 1.7 g;
Washing a bundle with 2% aqueous solution of sodium polyoxyethylene(3) laurylether sulfate, and then rinsing it with streaming tap water; and then
Drying it in the air to provide a hair bundle;
Dipping said hair bundle for 30 minutes in a bleaching agent, a mixture of 6% aqueous hydroperoxide and 2 % aqueous ammonia in the weight ratio of 1:1,
Rinsing it with streaming tap water,
Dipping said hair bundle for 5 minutes in a buffer solution of 1 M citric acid and 0.2 M disodium hydrogenphosphate (pH3),
Rinsing it with streaming tap water,
Drying it at room temperature in the air,
Repeating above procedure 5 times,
Dyeing hair with a commercially available oxidation type hair coloring agent for black,
Getting the dye hair bundle wet with hot water at 40 °C, Coating 3 g of the hair treatment agent obtained in Example 1 or Comparative examples 1 or 2 on the hair bundle and spreading and permeating them thoroughly,
Leaving it on a wrap film in a thermostat of 40 °C for 10 minutes,
Rinsing it with hot water, and
Drying it at room temperature in the air.

By using the hair bundles thus prepared, the properties of gloss, springness, combability and manageability of hair were sensory evaluated by 10 sensory-panelists in rating the properties as follows: best (point 2), second best (point 1) and worse (point 0); and then the average of rated points was defined as the evaluation value.

Then, the bundles of hair evaluated above were dipped in 200 ml of 1% (effective content) aqueous sodium polyoxyethylene(3) laurylether sulfate for 10 minutes, rinsed with streaming tap water, and then dried at room temperature in the air. After this procedure was repeated 10 times, the dyeing power of dyed hair were visually evaluated by 10 sensory-panelists in rating the properties as follows: deepest, (point 2), second deepest (point 1) and faint (point 0); and then the average of rated points was defined as the evaluation value for discoloration of dyed hair.

The results of evaluations are shown in Table 2 in terms of averaged value of ten panelists.

**Table 2**

| | | Example | Comparative example | |
|---|---|---|---|---|
| | | 1 | 1 | 2 |
| Treated hair: | | | | |
| | Gloss | 1.8 | 1.0 | 0.2 |
| | Springness | 1.7 | 0.8 | 0.5 |
| | Combability | 1.9 | 0.7 | 0.4 |
| | Manageability | 1.5 | 1.0 | 0.5 |
| Depth of color of dyed hair | | 1.8 | 0.7 | 0.5 |

As shown in Table 2, the evaluated values of the hair treated by the hair treatment agent of Example 1 are higher in every evaluation item such as gloss, springness, combability and manageability of hair in comparison with the hairs treated by the hair treatment agent of Comparative Examples 1 and 2. Therefore, the hair treatment agent of Example 1 blended with N-[2-hydroxy-3-(3'-methyldihydroxysilyl)propoxy]propyl hydrolyzed sericin-dimethyldiethoxysilane-octyltriethoxysilane copolymer composition clearly exerts excellent effects imparting gloss and springness to hairs, improving combability and manageability of hairs than the hair treatment agent of Comparative example 1 blended with polyoxyethylene/methylpolysiloxane copolymer and of Comparative example 2 not blended with a silylated peptide-silane compound, etc.

In the comparison of color depth of the dyed hair, almost all panelists answered that the color of hair treated with the hair treatment agent of Example 1 was deepest. This result shows that the hair treatment agent of Example 1 blended with N-[2-hydroxy-3-(3'-methyldihydroxysilyl)propoxy]propyl hydrolyzed sericin-dimethyldiethoxysilane-octyltriethoxysilane copolymer composition clearly exerts excellent effects suppressing discoloration by shampooing dyed hairs.

### Example 2 and Comparative Example 3

Two kinds of hair creams shown in Table 3 were prepared. After applying them on dyed hairs respectively, gloss, springness, combability and manageability of the treated hairs were evaluated.

In Example 2, N-[2-hydroxy-3-(3'-methyldihydroxysilyl)propoxy]propyl hydrolyzed sericin-dimethyldiethoxysilane-octyltriethoxysilane copolymer composition produced in Production Example 1, using a silylated peptide-silane compound copolymer composition, was used. In Comparative Example 3, in place of a silylated peptide-silane compound copolymer composition, poly(oxyethylene/oxipropylene)/methylpolysiloxane copolymer, a polyether modified silicone, was used.

**Table 3**

| | Example | Comparative example |
|---|---|---|
| | 2 | 3 |
| Silylated peptide-silane compound copolymer composition (70%) of Production Example 1 | 0.1 | 0 |
| Poly(oxyethylene/oxipropyl ene)/methylpolysiloxane copolymer*5 | 0 | 0.1 |
| Paraffin wax | 10.0 | 10.0 |
| Cetanol | 2.5 | 2.5 |
| Stearic acid | 4.5 | 4.5 |
| Glyceryl monoisostearate *6 | 0.5 | 0.5 |
| 2-ethylhexyl palmitate *7 | 15.0 | 15.0 |
| Liquid paraffin#350 | 15.0 | 15.0 |
| Triethanol amine | 1.8 | 1.8 |
| Propylene glycol | 1.0 | 1.0 |
| (Dihydroxymethylsily propoxy)hydroxyl propyl hydrolyzed wheat protein (20%) *8 | 0.1 | 0.1 |
| Presevative | Appropriate amount | Appropriate amount |
| Sterilized ion-exchange water | Volume adjustable totally 100 part | Volume adjustable totally 100 part |

| | | |
|---|---|---|
| *5: SH3749 (trade name), manufactured by Dow Corning Toray Silicone Co.,Ltd. *6: Ayacol GMIS (trade name), manufactured by SEIWA KASEI Co., Ltd. *7: Matlube 2EHP (trade name), manufactured by SEIWA KASEI Co., Ltd. *8: Promois WG-SIG (trade name) , manufactured by SEIWA KASEI Co., Ltd. | | |

Ten female panelists treated their hairs, each of which had been treated at least once by any of chemical treatments selected from bleaching, dyeing and permanent waving, with the hair cream of Comparative example 3 for the first 7 days and with the hair cream of Example 2 for the next 7 days, and comparison was carried out.

The treatment of hair with the cream was carried out by putting an appropriate amount (which varies depending on the amount of hairs) of hair cream on the palm of a hand, coating the cream with hand on hair, particularly on severely damaged hair tip, as a manner to rub the cream in the hair, and drying it with a hair dryer.

After the 14 days (that is, after completion of 7 days for use of the cream of Example 2 ) , they answered, regarding gloss, springness, combability and manageability of hair, which of creams of Example 2 and Comparative Example 3 is better or both are not different (, or almost same).

The results are shown in Table 4 in the numbers of the panelists who answered as Example 2 being better, who answered as Comparative Example 3 being better and who answered as there being no difference.

**Table 4**

| | | Number of persons who answered "Example 2 is better" | Number of persons who answered "Comparative example 3 is better" | Number of persons who answered "Cannot tell difference" |
|---|---|---|---|---|
| Treated hair: | | | | |
| | Gloss | 8 | 2 | 0 |
| | Springness | 8 | 2 | 0 |
| | Combability | 9 | 1 | 0 |
| | Manageability | 7 | 3 | 0 |

As shown in Table 4, the largest number of panelists answered that the hair cream of Example 2 blended with N-[2-hydroxy-3-(3'-methyldihydroxysilyl)propoxy]propyl hydrolyzed sericin-dimethyldiethoxysilane-octyltriethoxysilane copolymer composition is better than the hair cream of Comparative example 3 blended with poly(oxyethylene/oxipropylene)/methylpolysiloxane copolymer in every evaluation item. Particularly, almost all panelists who answered "Example 2 is better." told that unnatural force becomes not to be necessary on brushing due to the improvement of combability of hair tip. This indicates that N-[2-hydroxy-3-(3'-methyldihydroxysilyl)propoxy]propyl hydrolyzed sericin-dimethyldiethoxysilane-octyltriethoxysilane copolymer composition gives effect preventing formation of split hair. These results clearly show that N-[2-hydroxy-3-(3'-methyldihydroxysilyl)propoxy]propyl hydrolyzed sericin-dimethyldiethoxysilane-octyltriethoxysilane copolymer composition has excellent effect improving gloss, springness, combability and manageablity to used hairs and preventing formation of split hair from damaged hair.

### Example 3 and Comparative Examples 4 and 5

Three kinds of hair rinses shown in Table 5 were prepared. After applying them on damaged hairs respectively, and gloss, springness, smoothness, combability and manageability of the hairs were evaluated.

In Example 3, N-[2-hydroxy-3-(3'-methyldihydroxysilyl)propoxy]propyl hydrolyzed collagen-dimethyldiethoxysilane-octyltriethoxysilane copolymer composition produced in Production Example 2 was used. In Comparative Example 4, in place of a silylated peptide-silane compound copolymer composition, polyoxyethylene/methylpolysiloxane copolymer, a polyether modified silicone, was used. In Comparative Example 5, neither a silylated peptide-silane compound copolymer composition nor a silicone were used.

**Table 5**

| | Example | Comparative example | |
|---|---|---|---|
| | 3 | 4 | 5 |
| Silylated peptide-silane compound copolymer composition (70%) of Production Example 2 | 0.5 | 0 | 0 |
| Polyoxyethylene/methyl polysiloxane copolymer*9 | 0 | 0.5 | 0 |
| Diethyl aminoethylamide stearate *10 | 3.0 | 3.0 | 3.0 |
| Ethylene glycol distearate | 4.0 | 4.0 | 4.0 |
| Diglyceryl monoisostearate *11 | 1.5 | 1.5 | 1.5 |
| Lipophilic glyceryl monostearate | 0.5 | 0.5 | 0.5 |
| Behenyl alcohol | 6.0 | 6.0 | 6.0 |
| Cetyl trimethyl ammonium chloride(29%) | 3.5 | 3.5 | 3.5 |
| Distearyl dimethyl ammonium chloride(75%) | 1.5 | 1.5 | 1.5 |
| Propylene glycol | 3.0 | 3.0 | 3.0 |
| Piroctone olamine*12 | 0.3 | 0.3 | 0.3 |
| Hydrolyzed collagen (30%) *13 | 0.2 | 0.2 | 0.2 |
| Edetate disodium | Appropriate amount | Appropriate amount | Appropriate amount |
| Presevative | Appropriate amount | Appropriate amount | Appropriate amount |
| Sterilized ion-exchange water | Volume adjustable totally 100 part | Volume adjustable totally 100 part | Volume adjustable totally 100 part |

| | | | |
|---|---|---|---|
| *9 SH3775 M (trade name ) , manufactured by Dow Corning Toray Silicone Co.,Ltd. *10: Ayacol amineamide 50E (trade name), manufactured by SEIWA KASEI Co., Ltd. *11: Ayacol GMIS (trade name), manufactured by SEIWA KASEI Co., Ltd. *12: Octopirox (trade name) , manufactured by Lion Co . , Ltd. *13: Promois WU-32R (trade name), manufactured by SEIWA KASEI Co., Ltd. | | | |

Three bundles of damaged hairs subjected to bleaching according to the same procedure as in Example 1 were prepared and the following hair rinse treatment was carried out.

More concrete steps are as follows:
Washing one of the bundle of damaged hair with 4% aqueous solution of sodium polyoxyethylene (3) laurylether sulfate, and then rinsing it with streaming tap water; and then
Wiping out water lightly in the bundle with a towel,
Coating 2 g of the hair rinse obtained in Example 3 or Comparative examples 4 or 5 on the hair bundle and spreading and permeating them thoroughly,
Leaving it on a wrap film for 5 minutes,
Rinsing it with hot water,
Drying it at room temperature in the air, and
Repeating above procedure 3 times.

Thus obtained hair samples were subjected to the following evaluation.

The hair smoothness of the treated hair was evaluated by measuring the friction force of the treated hair surface with the friction tester (a trade name of KES-SE, made by Katotech Co., Ltd.) under the conditions of humidity of 40 ± 1% and temperature 22 ± 1 °C.

The smoothness ( , or roughness) is represented in said tester with the value of average deviation of friction coefficient sensed by a friction sensor traversing the specified distance on the sample surface, wherein the unit of said value is dimensionless and the value indicates "more smoothness" when the value becomes smaller. In the present test, measurement was carried out10 times per one sample and the average of obtained values was defined as the measurement value.

Then, by using the same bundles of hair, the properties of gloss, springness, smoothness, combability and manageability of hair were sensory evaluated by 10 panelists according to the same rating as in Example 1.

The results of evaluations are shown in Table 6 in terms of averaged value of ten panelists.

**Table 6**

| | | Example | Comparative example | |
|---|---|---|---|---|
| | | 3 | 4 | 5 |
| Hair smoothness (The average deviation of friction coefficient) | | 0.0038 | 0.0045 | 0.0048 |
| Treated hair: | | | | |
| | Gloss | 1.7 | 0.9 | 0.4 |
| | springness | 1.6 | 0.8 | 0.6 |
| | Smoothness | 1.6 | 1.0 | 0.4 |
| | Combability | 1.9 | 0.7 | 0.4 |
| | Manageability | 1.5 | 0.8 | 0.7 |

As shown in Table 6, the average deviation value of friction coefficient, which indicates hair smoothness, of the hair treated by the hair rinse of Example 3 is the smallest; and said value is 84.4% of that of the hair treated by the hair rinse of Comparative example 4, and 79.2% of that of the hair treated by the hair rinse of Comparative Example 5.
According this result, N-[2-hydroxy-3-(3'-methyldihydroxysilyl)propoxy]propyl hydrolyzed collagen-dimethyldiethoxysilane-octyltriethoxysilane copolymer composition, which is blended in Example 3, clearly exerts effects to be sorbed on and makes smooth the hair surface.

Moreover, according to the sensory evaluation, the evaluated values of the hair treated by the hair rinse of Example 3 are higher in every evaluation item such as gloss, springness, smoothness, combability and manageability of hair in comparison with the hairs treated by the hair rinse of Comparative Example 4 and 5. Therefore, N-[2-hydroxy-3-(3'-methyldihydroxysilyl)propoxy]propyl hydrolyzed collagen-dimethyldiethoxysilane-octyltriethoxysilane copolymer composition clearly exerts excellent effects imparting gloss and springness to hairs, improving smoothness, combability and manageability of hairs.

### Example 4 and Comparative Example 6

Two kinds of hair conditioners shown in Table 7 were prepared. After applying them on dyed hairs respectively, and gloss, springness, smoothness, combability, manageability of the hairs and degree of discoloration of dyed hairs were evaluated.

In Example 4, N-[2-hydroxy-3-(3'-methyldihydroxysilyl)propoxy]propyl hydrolyzed silk-dimethyldiethoxysilane-octyltriethoxysilane copolymer composition produced in Production Example 3 was used. In Comparative Example 6, in place of a silylated peptide-silane compound copolymer composition,
polyoxyethylene/methylpolysiloxane copolymer, a polyether modified silicone, was used.

**Table 7**

| | Example | Comparative example |
|---|---|---|
| | 4 | 6 |
| Silylated peptide-silane compound copolymer composition (70%) of Production Example 3 | 1.0 | 0 |
| Polyoxyethylene/methylpoly siloxane copolymer*14 | 0 | 1.0 |
| Decylalcohol-polyethyleneg lycol-polyurethane copolymer/propylene glycol/water mixture*15 | 2.0 | 2.0 |
| Behenyl alcohol | 2.0 | 2.0 |
| Stearyl alcohol | 1.5 | 1.5 |
| Isopropyl isostearate*16 | 3.5 | 3.5 |
| Lipophilic glyceryl monostearate | 0.8 | 0.8 |
| Diethylaminoethylamide stearate*17 | 1.0 | 1.0 |
| Polyoxyethylene (200) monostearate | 1.0 | 1.0 |
| Cocodimonium hydroxypropyl hydrolyzed Keratin (30%)*18 | 0.1 | 0.1 |
| Citric acid | 0.1 | 0.1 |
| Presevative | Appropriate amount | Appropriate amount |
| Sterilized ion-exchange water | Volume adjustable totally 100 part | Volume adjustable totally 100 part |

| | | |
|---|---|---|
| *14 SH3775 M (trade name) , manufactured by Dow Corning Toray Silicone Co.,Ltd. *15: Aculyn 44 (trade name), manufactured by Roam & Haas Co., Ltd. *16: Matlube I·I (trade name) , manufactured by SEIWA KASEI Co., Ltd. *17: Ayacol amineamide 50E(trade name), manufactured by SEIWA KASEI Co., Ltd. *18: Promois WK-HCAQ (trade name), manufactured by SEIWA KASEI Co., Ltd. | | |

Two bundles of damaged hairs subjected to bleaching 5 times according to the same procedure as in Example 1, followed by dyed were prepared and, then, the following hair conditioning treatment was carried out.

More concrete steps are as follows:
Washing one of the bundle of damaged hair with 4% aqueous solution of polyoxyethylene(3)lauryleter sodium sulfate, and then rinsing it with streaming tap water; and then
Wiping out water lightly in the bundle with a towel,
Coating 2 g of the hair conditioner obtained in Example 4 or Comparative example 6 on the hair bundle and spreading and permeating them thoroughly,
Leaving it on a wrap film for 5 minutes,
Rinsing it with hot water,
Drying it at room temperature in the air, and
Repeating above procedure 10 times.

Thus obtained hair samples were sensory evaluated by 10 panelists regarding gloss, springness, smoothness, combability and manageability of hair, and degree of discoloration of hair, and they answered which of hair conditioner of Example 4 and Comparative Example 6 is better or both are not different (, or almost same).

The results are shown in Table 8 in the numbers of the panelists who answered as Example 4 being better, who answered as Comparative Example 6 being better and who answered as there being no difference.

**Table 8**

| | | Number of persons who answered "Example 4 is better" | Number of persons who answered "Comparative example 6 is better" | Number of persons who answered "Cannot tell difference" |
|---|---|---|---|---|
| Treated hair: | | | | |
| | Gloss | 8 | 0 | 2 |
| | Springness | 7 | 0 | 3 |
| | Smoothness | 6 | 0 | 4 |
| | Combability | 9 | 0 | 1 |
| | Manageability | 7 | 0 | 3 |
| | Discoloration | 9 | 0 | 1 |

As shown in Table 8, the evaluated values of the hair treated by the hair conditioner of Example 4 are higher in every evaluation item of gloss, springness, smoothness, combability and manageability of hair in comparison with the hair treated by the hair conditioner of Comparative Example 6. Regarding evaluation of discoloration, almost all panelists answered that hair treated in Example 4 had deeper color than hair treated in Comparative example 6. Therefore, N-[2-hydroxy-3-(3'-methyldihydroxysilyl)propoxy]propyl hydrolyzed silk-dimethyldiethoxysilane-octyltriethoxysilane copolymer composition clearly exerts excellent effects imparting gloss and springness to hairs, improving smoothness, combability and manageability of hairs and suppressing discoloration of dyed hair by shampooing.

### Example 5 and Comparative Example 7

A hair wax was prepared using N-[2-hydroxy-3-(3'-methyldihydroxysilyl)propoxy]propyl hydrolyzed silk-dimethyldiethoxysilane-octyltriethoxysilane copolymer composition produced in Production Example 3.

**Table 9-1**

| | Example 5 | Comparative Example 7 |
|---|---|---|
| Silylated peptide-silane compound copolymer composition (70%) of Production Example 3 | 0.05 | - |
| Candelilla wax | 5.0 | 5.0 |
| Paraffin wax | 15.0 | 15.0 |
| Polyoxyethylene(10) oleylether | 3.0 | 3.0 |
| Cetanol | 0.5 | 0.5 |
| Liquid paraffin | 10.0 | 10.0 |
| Squalane | 10.0 | 10.0 |
| 1,3-Butyleneglycol | 3.0 | 3.0 |
| Sodium hydroxide | Amount to adjust pH6.8 | Amount to adjust pH6.8 |
| Sterilized ion-exchange water | Volume adjustable totally 100 part | Volume adjustable totally 100 part |

Ten panelists used the two waxes obtained above on their hair, and, then, sensory evaluated about gloss, springness, and manageability.

The sensory evaluation test was carried by the half-head method, in which the panelists combed their hair in two sides, that is, right and left sides, and then treated one of sides with one of the two waxes and the rest side with the other wax.

Treatment with the hair wax was carried out by coating an appropriate amount (which varies depending on the amount and length of hairs) of hair wax on the hair, and rubbing and spreading the wax in whole hair.

Gloss, springness, combability and manageability of hair were sensory evaluated by 10 panelists in rating the properties as follows: better (point 1) and worse (point 0) ; and then total of rated points by 10 panelists was defined as the evaluation value and shown in Table 9-2.

**Table 9-2**

| | | Example 5 | Comparative example 7 |
|---|---|---|---|
| Treated hair: | | | |
| | Gloss | 10 | 0 |
| | Springness | 8 | 2 |
| | Manageability | 9 | 1 |

As shown in Table 9-2, the evaluated values of the hair treated by the hair wax of Example 5 are higher in every evaluation item of gloss, springness, and manageability of hair in comparison with the hair treated by the hair wax of Comparative Example 7. Therefore, N-[2-hydroxy-3-(3'-methyldihydroxysilyl)propoxy]propyl hydrolyzed silk-dimethyldiethoxysilane-octyltriethoxysilane copolymer composition contained in a hair wax clearly exerts excellent effects imparting gloss and springness to hairs, and improving manageability of hairs.

### Example 6 and Comparative Example 8

A hair spray undiluted solution was prepared using N-[2-hydroxy-3-(3'-methyldihydroxysilyl)propoxy]propyl hydrolyzed collagen-dimethyldiethoxysilane-octyltriethoxysilane copolymer composition produced in Production Example 2, and the prepared undiluted solution was mixed with dimethylether in a weight ratio of 50:50 to prepare hair spray.

**Table 10-1**

| | Example 6 | Comparative Example 8 |
|---|---|---|
| Silylated peptide-silane compound copolymer composition (70%) of Production Example 2 | 0.7 | - |
| Acrylic resin alkanolamine solution (50%) | 1.0 | 1.0 |
| Cetanol | 0.1 | 0.1 |
| Methyl polysiloxane*19 | 0.5 | 0.5 |
| Polyoxyethylene(25)cetylether | 0.8 | 0.8 |
| purfume | Appropriate amount | Appropriate amount |
| Ethanol | Volume adjustable totally 100 part | Volume adjustable totally 100 part |

| | | |
|---|---|---|
| *19 SH200C-10cs (trade name), manufactured by Dow Corning Toray Silicone Co.,Ltd. | | |

Ten panelists sprayed uniformly the hair sprays obtained above on their hair, and, then, evaluated about gloss, springness, and manageability according to the same rating as in Example 5; and then total of rated points by 10 panelists was defined as the evaluation value and shown in Table 10-2.

**Table 10-2**

| | | Example 6 | Comparative example 8 |
|---|---|---|---|
| Treated hair: | | | |
| | Gloss | 9 | 1 |
| | Springness | 8 | 2 |
| | Manageability | 9 | 1 |

As shown in Table 10-2, the evaluated values of the hair treated by the hair spray of Example 6 are higher in every evaluation item of gloss, springness, and manageability of hair in comparison with the hair treated by the hair spray of Comparative Example 8. Therefore, N-[2-hydroxy-3-(3'-methyldihydroxysilyl)propoxy]propyl hydrolyzed collagen-dimethyldiethoxysilane-octyltriethoxysilane copolymer composition contained in a hair spray clearly exerts excellent effects imparting gloss and springness to hairs, and improving manageability of hairs.

### Example 7 and Comparative Examples 9 and 10

Three kinds of shampoo compositions shown in Table 11 were prepared. After shampooing hair 10 times by using each shampoo composition respectively, the hair smoothness was evaluated and the properties of gloss, combability and manageability of hair were also evaluated.

In Example 7, N-[2-hydroxy-3-(3'-methyldihydroxysilyl)propoxy]propyl hydrolyzed sericin-dimethyldiethoxysilane-octyltriethoxysilane copolymer composition produced in Production Example 1 was used for a shampoo. In Comparative Example 9, in place of a silylated peptide-silane compound copolymer composition, a silylated hydrolyzed sericin having about 500 of the number average molecular weight in its peptide portion was used for a shampoo; in Comparative Example 10, neither a silylated peptide-silane compound copolymer composition nor a silylated peptide were used for a shampoo.

**Table 11**

| | Example | Comparative example | |
|---|---|---|---|
| | 7 | 9 | 10 |
| Silylated peptide-silane compound copolymer composition (70%) of Production Example 1 | 1.0 | 0 | 0 |
| Silylated hydrolyzed sericin (20%) | 0 | 5.0 | 0 |
| Potassium (palm oil fatty acid)hydrolyzed collagen (35%) (atradenameofPromois ECP, manufactured by SEIWA KASEI Co., Ltd.) | 50.0 | 50.0 | 50.0 |
| (Palm oil fatty acid)diethanolamide | 3.5 | 3.5 | 3.5 |
| Paraoxybenzoic acid ester-phenoxyethanol mixture (a trade name of Seisept H, manufactured by SEIWA KASEI Co., Ltd.) | 0.5 | 0.5 | 0.5 |
| Perfume | Appropriate amount | Appropriate amount | Appropriate amount |
| Sterilized ion-exchange water | Volume adjustable totally 100 part | Volume adjustable totally 100 part | Volume adjustable totally 100 part |

The hairs were treated for evaluation by following steps:
Preparing three bundles of hair, each bundle being the length of 15 cm and the weight of 1.5 g;
Washing a bundle with 2% aqueous solution of sodium polyoxyethylene(3) laurylether sulfate, and then rinsing it with streaming tap water; and then
Drying it with a hair dryer to provide a hair bundle for shampoo treatment;
Getting wet said hair bundle for shampoo treatment with hot water;
Washing each hair bundle with 1 g of each of shampoos prepared in Example 7, Comparative Example 9 and 10 respectively;
Rinsing it with streaming hot water; and then drying it with a hair dryer;
Repeating above procedures, which are shampooing, rinsing and drying with hair dryer, in 10 cycles; and then
Each of hair bundles was served to the hair smoothness evaluation test.

The hair smoothness was evaluated by measuring the friction force of the treated hair surface with the friction feeling tester (a trade name of KES-SE, made by Katotech Co., Ltd.) under the conditions of humidity of 40 ± 1% and temperature 22 ± 1 °C.

The smoothness ( , or roughness) is represented in said tester with the value of average deviation of friction coefficient sensed by a friction sensor traversing the specified distance on the sample surface, wherein the unit of said value is dimensionless and the value indicates "more smoothness" when the value becomes smaller. In the present test, measurement was carried out10 times per one sample and the average of obtained values was defined as the measurement value.

Then, by using the same bundles of hair, the properties of gloss, combability and manageability of hair were sensory evaluated by 10 sensory-panelists in rating the properties as follows: best (point 2), second best (point 1) and worse (point 0); and then the average of rated points was defined as the evaluation value. The results of evaluations are shown in Table 12 in terms of averaged value.

**Table 12**

| | | Example | Comparative example | |
|---|---|---|---|---|
| | | 7 | 9 | 10 |
| Hair smoothness (The average deviation of friction coefficient) | | 0.0040 | 0.0045 | 0.0051 |
| | Treated hair: | | | |
| | Gloss | 1.6 | 1.4 | 0 |
| | Combability | 2.0 | 1.0 | 0 |
| | Manageability | 1.8 | 1.2 | 0 |

As shown in Table 12, the average deviation value of friction coefficient, which indicates hair smoothness, of the hair shampooed by the shampoo of Example 7 containing the silylated peptide-silane compound copolymer composition is the smallest; and said value is 88.9% of that of the hair shampooed by the shampoo of Comparative Example 9 blended with the silylated peptide, and 78.4% of that of the hair shampooed by the shampoo of Comparative Example 10 containing neither the silylated peptide-silane compound copolymer composition nor the silylated peptide. According this result, N-[2-hydroxy-3-(3'-methyldihydroxysilyl)propoxy]propyl hydrolyzed sericin-dimethyldiethoxysilane-octyltriethoxysilane copolymer composition, which is blended in Example 7, clearly exerts effects to be sorbed on and makes smooth the hair surface when said copolymer composition is contained in a shampoo.

Moreover, according to the sensory evaluation, the evaluated values of the hair shampooed by the shampoo of Example 7 are higher in every evaluation item such as gloss, combability and manageability of hair in comparison with the hairs shampooed by the shampoos of Comparative Example 9 and 10. Therefore, the silylated peptide-silane compound copolymer composition blended in a shampoo of Example 7 clearly exerts excellent effects imparting gloss to hairs, improving combability and manageability of hairs after being shampooed.

### Example 8 and Comparative Example 11

Two kinds of shampoo compositions shown in Table 13 were prepared. After shampooing hairs with each shampoo composition respectively, the gloss, smoothness, combability and manageability of hair for shampooed hair were sensory evaluated.

In Example 8, N-[2-hydroxy-3-(3'-methyldihydroxysilyl)propoxy]propyl hydrolyzed collagen-dimethyldiethoxysilane-octyltriethoxysilane copolymer composition of the silylated peptide-silane compound copolymer composition produced in Production Example 2 was used. In Comparative Example 11, in place of a silylated peptide-silane compound copolymer composition, a silylated hydrolyzed collagen having about 500 of the number average molecular weight in its peptide portion was used.

**Table 13**

| | Example | Comparative example |
|---|---|---|
| | 8 | 11 |
| Silylated hydrolyzed peptide-silane compound copolymer composition (70%) of Production Example 2 | 0.2 | 0 |
| Silylated hydrolyzed collagen (20%) | 0 | 0.7 |
| Monosodium N-lauroyl-L-glutaminate | 15.0 | 15.0 |
| Coconut fatty acid diethanolamide | 3.5 | 3.5 |
| Stearyltrimethyl ammonium chloride (30%) | 3.3 | 3.3 |
| Paraoxybenzoic acid ester-phenoxyethanol mixture (a trade name of Seisept H, manufactured by SEIWA KASEI Co. , Ltd.) | 0.5 | 0.5 |
| Perfume | Appropriate amount | Appropriate amount |
| Sterilized ion-exchange water | Volume adjustable totally 100 part | Volume adjustable totally 100 part |

The sensory evaluation test for the above described shampoo was carried by the half-head method, in which 10 sensory-panelists (consisting of 7 females and 3 males) combed their hair in two sides, that is, right and left sides, and then shampooed one of sides with the shampoo of Example 8 and the rest side with the shampoo of Comparative Example 11. Although the amount of shampoo used was different depending on the volume and length of their hair, they shampooed once a day for seven days, then evaluated the properties of gloss, smoothness, combability and manageability of hair about that which of shampoo of Example 8 and Comparative Example 11 is better or both are not different (, or almost same).

The result is shown in Table 14 in the numbers of the panelists who answered as Example 8 being better, who answered as Comparative Example 11 being better and who answered as there being no difference.

**Table 14**

| | | Numbers answered as Example 8 better | Numbers answered as Comparative example 11 better | Numbers answered as no difference |
|---|---|---|---|---|
| For shampooed hair | | | | |
| | Gloss | 9 | 0 | 1 |
| | Smoothness | 10 | 0 | 0 |
| | Combability | 10 | 0 | 0 |
| | Manageability | 7 | 0 | 3 |

As shown in Table 14, the number of the panelists who answered as Example 8 being better is largest in every evaluation item, particularly, on the properties of smoothness and combability, every panelist answered as Example 8 being better. According this result, N-[2-hydroxy-3-(3'-methyldihydroxysilyl)propoxy]propyl hydrolyzed collagen-dimethyldiethoxysilane-octyltriethoxysilane copolymer composition is clearly excellent in the effects to provide smoothness and combability to hair, moreover clearly exerts effects of imparting gloss to hairs and imparting manageability of hairs after hair being shampooed.

### Example 9 and Comparative Example 12 and 13

Three kinds of shampoo compositions shown in Table 15 were prepared. After shampooing hairs in 10 cycles with each shampoo composition respectively, the hair smoothness was evaluated and the properties of gloss, combability and manageability of hair were also evaluated.

In Example 9, N-[2-hydroxy-3-(3'-methyldihydroxysilyl)propoxy]propyl hydrolyzed silk-dimethyldiethoxysilane-octyltriethoxysilane copolymer composition of the silylated peptide-silane compound copolymer composition produced in Production Example 3 was used for a shampoo. In Comparative Example 12, in place of a silylated peptide-silane compound copolymer composition, a silylated hydrolyzed silk having about 600 of the number average molecular weight in its peptide portion was used for a shampoo; in Comparative Example 13, neither a silylated peptide-silane compound copolymer composition nor a silylated peptide were used for a shampoo.

**Table 15**

| | Example | Comparative example | |
|---|---|---|---|
| | 9 | 12 | 13 |
| Silylated peptide-silane compound copolymer composition (70%) of Production Example 3 | 0.1 | 0 | 0 |
| Silylated hydrolyzed silk (20%) | 0 | 0.4 | 0 |
| Cocoylamidepropyldimethyl glycine (30%) | 17.1 | 17.1 | 17.1 |
| Polyoxyethylene(2) dodecyleter sulfate | 8.3 | 8.3 | 8.3 |
| Polyoxyethylene(100EO) sorbitanmonolaurate | 7.5 | 7.5 | 7.5 |
| Paraoxybenzoic acid ester-phenoxyethanol mixture (a trade name of Seisept H, manufactured by SEIWA KASEI Co., Ltd.) | 0.5 | 0.5 | 0.5 |
| Perfume | Appropriate amount | Appropriate amount | Appropriate amount |
| Sterilized ion-exchange water | Volume adjustable totally 100 part | Volume adjustable totally 100 part | Volume adjustable totally 100 part |

By using shampoos obtained by above Example 9, Comparative Example 12 and 13 respectively, each bundle of hair having the length of 15 cm and the weight of 1.5 g was washed with 0.5 g of shampoo and hot water, and then was rinsed with hot water, and then was dried with a hair dryer. After repeating this procedure in 10 cycles, with the same manner carried in Example 7, the smoothness of hair surface was measured with the friction feeling tester, and then the properties of gloss, combability and manageability of hair were sensory evaluated by 10 female sensory-panelists under same rating rule applied in Example 7. The results of those evaluations are shown in Table 16 in average values.

**Table 16**

| | | Example | Comparative example | |
|---|---|---|---|---|
| | | 9 | 12 | 13 |
| Hair smoothness (The average deviation of friction coefficient) | | 0.0043 | 0.0046 | 0.0050 |
| Treated hair: | | | | |
| | Gloss | 1.7 | 1.3 | 0 |
| | Combability | 1.9 | 1.1 | 0 |
| | Manageability | 1.8 | 1.2 | 0 |

As shown in Table 16, the average deviation value of friction coefficient, which indicates hair smoothness, of the hair shampooed by the shampoo of Example 9 containing the silylated peptide-silane compound copolymer composition is the smallest; and said value is 93.5% of that of the hair shampooed by the shampoo of Comparative Example 12 containing the silylated peptide, and 82.7% of that of the hair shampooed by the shampoo of Comparative Example 13 containing neither the silylated peptide-silane compound copolymer composition nor the silylated peptide. According this result, N-[2-hydroxy-3-(3'-methyldihydroxysilyl)propoxy]propyl hydrolyzed silk-dimethyldiethoxysilane-octyltriethoxysilane copolymer composition, which is blended in Example 9, clearly exerts effects to be sorbed on and makes smooth the hair surface when said copolymer composition is contained in a shampoo.

Moreover, according to the sensory evaluation, the evaluated values of the hair shampooed by the shampoo of Example 9 are higher in every evaluation item such as gloss, combability and manageability of hair in comparison with the hairs shampooed by the shampoos of Comparative Example 12 and 13. Therefore, the silylated peptide-silane compound copolymer composition blended in a shampoo of Example 9 clearly exerts excellent effects imparting gloss to hairs, improving combability of hairs and imparting manageability of hairs after being shampooed.

### Example 10 and Comparative Example 14 and 15

First compositions of hair coloring agents (oxidation type) having composition as shown in Table 17 were prepared, and evaluated about dyeability, and gloss, springness, combability and shampoo fastness of the dyed hairs.

In Example 10, N-[2-hydroxy-3-(3'-methyldihydroxysilyl)propoxy]propyl hydrolyzed sericin-dimethyldiethoxysilane-octyltriethoxysilane copolymer composition produced in Production Example 1 was used. In Comparative Example 14, in place of a silylated peptide-silane compound copolymer composition, aminomethylaminopropylsiloxane/dimethylsiloxane copolymer emulsion (SM8702C (trade name), manufactured by Dow Corning Toray Silicone Co.,Ltd.), an amino modified silicone, was used; in Comparative Example 15 (Control, blank), neither a silylated peptide-silane compound copolymer composition nor a silicone were used.

**Table 17**

| | Example | Comparative example | |
|---|---|---|---|
| | 10 | 14 | 15 |
| Silylated peptide-silane compound copolymer composition (70%) of Production Example 1 | 3.0 | 0 | 0 |
| Amino modified silicone (40%) | 0 | 5.3 | 0 |
| p-phenylenediamine | 0.8 | 0.8 | 0.8 |
| p-aminophenol | 0.2 | 0.2 | 0.2 |
| o-aminophenol | 1.0 | 1.0 | 1.0 |
| Resolcinol | 1.6 | 1.6 | 1.6 |
| Isopropanol | 9.0 | 9.0 | 9.0 |
| Propyleneglycol | 5.5 | 5.5 | 5.5 |
| Oleic acid | 15.0 | 15.0 | 15.0 |
| Aqueous ammonia (28%) | 10.0 | 10.0 | 10.0 |
| Sodium sulfite | 0.1 | 0.1 | 0.1 |
| Oleyl alcohol | 10.0 | 10.0 | 10.0 |
| Edetate disodium | 0.4 | 0.4 | 0.4 |
| Bis-2-hydroxyethyl-sorbitaneamine | 9.4 | 9.4 | 9.4 |
| Hydroxyethl stearylamide | 6.0 | 6.0 | 6.0 |
| Sterilized ion-exchange water | Volume adjustable totally 100 part | Volume adjustable totally 100 part | Volume adjustable totally 100 part |

A second composition having composition as shown in Table 17-2 was prepared for the first composition of Example 10 and Comparative examples 14 and 15.

**Table 17-2**

| | |
|---|---|
| Stearic acid | 1.0 |
| Glycerin monostearate | 1.5 |
| Polyoxyethylene(5) oleyl ether | 1.0 |
| Hydrogen peroxide(35%) | 15.0 |
| Sterilized ion-exchange water | Volume adjustable totally 100 part |

The hairs were treated for evaluation by following steps:
Preparing three bundles of hair, each bundle being the length of 10 cm and the weight of 1 g;
Washing a bundle with 2% aqueous solution of polyoxyethylene(3)lauryleter sodium sulfate, and then rinsing it with water;
Dipping it for 5 minutes in 10 g (for each bundle) of solution prepared by mixing 6% aqueous hydrogenperoxide solution and 2% aqueous ammonia in a weight ratio of 1: 1 as bleaching in order to make the comparison of dyeing power of dyed hairs easier;
rinsing it with water;
Drying the hair; and then
Being subjected dyeing.

Dyeing was carried out by mixing same amounts of the first composition and the second composition, coating 2 g of the mixture on each hair bundle, then leaving the bundle for 15 minutes, rinsing it with hot water, and washing it with 2% aqueous solution of sodium polyoxyethylene(3) laurylether sulfate. After dyeing was completed, the hair bundle was dried with a hair dryer, and the obtained hair bundle was evaluated about uniform dyeability, gloss, springness and combability by ten panelists.

In the evaluation, Example 10 and Comparative example 14 were compared with Comparative example 15 (Control), and evaluated according to the following rating.

Rating
- +3:: Much better than Control
- +2:: Better than Control
- +1:: A little better than Control
- 0:: no difference was found with Control
- - 1:: A little worse than Control
- - 2:: Worse than Control
- - 3:: Much worse than Control

A bundle of dyed hair with the hair coloring agent of Example 10, Comparative example 14 or Comparative example 15 was washed with 2% aqueous solution of sodium polyoxyethylene(3) laurylether sulfate, rinsed with water and dried with a hair dryer. After repeating above procedure 20 times, the washed hair bundle was evaluated about depth of color after washing, gloss, springness and combability by ten panelists according to the same rating as for the above evaluation. The results indicate shampoo fastness and are shown in Table 18 in terms of averaged value of ten panelists.

**Table 18**

| | | Example | Comparative example |
|---|---|---|---|
| | | 10 | 14 |
| Dyed hair before washing: | | | |
| | Uniform dyeability | +2.3 | +1.6 |
| | Gloss | +2.4 | +1.5 |
| | Springness | +2.3 | +1.0 |
| | Combability | +2.2 | +0.6 |
| Dyed hair after washing: | | | |
| | Color Depth | +2.9 | +0.8 |
| | Gloss | +1.9 | +1.1 |
| | Springness | +2.3 | +0.6 |
| | Combability | +1.9 | +0.8 |

As shown in Table 18, the evaluated values of the hair dyed by the hair coloring agent of Example 10 are rated plus(+), that is, better than the hair dyed by the hair coloring agent of Comparative example 15 (Control), and are higher in every evaluation item in comparison with the hair dyed by the hair coloring agent of Comparative example 14. Therefore, N-[2-hydroxy-3-(3'-methyldihydroxysilyl)propoxy]propyl hydrolyzed sericin-dimethyldiethoxysilane-octyltriethoxysilane copolymer composition, blended with the hair coloring agent, clearly exerts excellent effects giving uniform dyeability, suppressing damage of hair on dyeing (good combability indicates this fact), and imparting gloss, springness and combability to hairs. On the other hand, the hair dyed by the hair coloring agent of Comparative example 14, containing an amino modified silicone in place of a silylated peptide-silane compound copolymer composition, does not show high evaluated values, that is, improvements from Control are small, in every evaluation item such as gloss, springness and combability of hair. From this result, it is contemplated that the silicone blended in the hair coloring agent, is hard to be sorbed by hair or the silicone is easily washed away by washing after dyeing and the sorption amount is little, and, as the result, the effect is not realized.

Even afterwashing 20 times with shampoo, the evaluated values of the hair dyed by the hair coloring agent of Example 10 still higher in every evaluation items such as color depth, gloss, springness and combability of hair in comparison with the hair dyed by the hair coloring agent of Comparative example 14. From this result, it is clear that silylated peptide-silane compound copolymer composition blended in the hair coloring agent of Example 10 improve shampoo fastness of dyed hair.

### Example 11 and Comparative Example 16 and 17

First compositions of hair coloring agents having composition as shown in Table 19 were prepared, and evaluated about uniform dyeability, and gloss, springness and combability of the dyed hairs.

In Example 11, N-[2-hydroxy-3-(3'-methyldihydroxysilyl)propoxy]propyl hydrolyzed collagen-dimethyldiethoxysilane-octyltriethoxysilane copolymer composition produced in Production Example 2 was used. In Comparative Example 16, in place of a silylated peptide-silane compound copolymer composition, dimethylpolysiloxane copolymer emulsion (SH200C-100cs (trade name), manufactured by Dow Corning Toray Silicone Co.,Ltd.), an amino modified silicone, was used; in Comparative Example 17 (Control, blank), neither a silylated peptide-silane compound copolymer composition nor a silicone were used.

**Table 19**

| | Example | Comparative example | |
|---|---|---|---|
| | 11 | 16 | 17 |
| Silylated peptide-silane compound copolymer composition (70%) of Production Example 2 | 5.0 | 0 | 0 |
| Dimethylpolysiloxane | 0 | 3.5 | 0 |
| Nitro-p-phenylenediamine | 0.4 | 0.4 | 0.4 |
| 4-amino-2-nitrophenol | 0.4 | 0.4 | 0.4 |
| o-aminophenol | 0.1 | 0.1 | 0.1 |
| Resolcinol | 0.1 | 0.1 | 0.1 |
| Polyoxyethylene(7)nonyl phenyl ether | 10.0 | 10.0 | 10.0 |
| Oleic acid | 5.0 | 5.0 | 5.0 |
| Oleyl alcohol | 10.0 | 10.0 | 10.0 |
| Propyleneglycol | 10.0 | 10.0 | 10.0 |
| Isopropanol | 10.0 | 10.0 | 10.0 |
| Ascorbic acid | 0.5 | 0.5 | 0.5 |
| Aqueous ammonia (28%) | To adjust pH 9.5 | To adjust pH 9.5 | To adjust pH 9.5 |
| Sterilized ion-exchange water | Volume adjustable totally 100 part | Volume adjustable totally 100 part | Volume adjustable totally 100 part |

Second compositions of hair coloring agents having composition as shown in Table 19-2 were prepared.

**Table 19-2**

| | Example | Comparative example | |
|---|---|---|---|
| | 11 | 16 | 17 |
| Hydrogen peroxide (35%) | 15.0 | 15.0 | 15.0 |
| Dimethylpolysiloxane | 0.1 | 0.1 | 0.1 |
| Nitro-p-phenylenediamine | 84.9 | 84.9 | 84.9 |

Three bundles of hair, each bundle being the length of 10 cm and the weight of 1 g, were prepared, and bleached according to the same manner as in Example 10. Then dyeing was carried out using the hair coloring agent of Example 11, Comparative example 16 or Comparative example 17.

Dyeing was carried out by mixing same amounts of the first composition and the second composition, coating 2 g of the mixture on each hair bundle, then leaving the bundle for 20 minutes, rinsing it with hot water, and washing it with 2% aqueous solution of sodium polyoxyethylene(3) laurylether sulfate. After dyeing was completed, the hair bundle was dried with a hair dryer, and the obtained hair bundle was evaluated about uniform dyeability, gloss, springness and combability by ten panelists according to the same rating as in Example 10.

Further, a bundle of dyed hair with the hair coloring agent of Example 11, Comparative example 16 or Comparative example 17 was washed with 2% aqueous solution of sodium polyoxyethylene(3) laurylether sulfate, rinsed with water and dried with a hair dryer. After repeating above procedure 20 times, the washed hair bundle was evaluated about depth of color after washing, gloss, springness and combability by ten panelists according to the same rating as in Example 10. The results indicate shampoo fastness and are shown in Table 20 in terms of averaged value of ten panelists.

**Table 20**

| | | Example | Comparative example |
|---|---|---|---|
| | | 11 | 16 |
| Dyed hair before washing: | | | |
| | Uniform dyeability | +2.3 | +1.3 |
| | Gloss | +2.4 | +1.1 |
| | Springness | +2.0 | +0.9 |
| | Combability | +2.8 | +0.5 |
| Dyed hair after washing: | | | |
| | Color Depth | +2.8 | +0.6 |
| | Gloss | +2.0 | +0.9 |
| | Springness | +2.1 | +0.5 |
| | Combability | +2.0 | +1.0 |

As shown in Table 20, the evaluated values of the hair dyed by the hair coloring agent of Example 11 blended with N-[2-hydroxy-3-(3'-methyldihydroxysilyl)propoxy]propyl hydrolyzed collagen-dimethyldiethoxysilane-octyltriethoxysilane copolymer composition are rated plus(+), that is, better than the hair dyed by the hair coloring agent of Comparative example 17 (Control), and are higher in every evaluation item in comparison with the hair dyed by the hair coloring agent of Comparative example 16 blended with an amino modified silicone. Therefore, the silylated peptide-silane compound copolymer composition blended in the hair coloring agent of Example 11 clearly exerts excellent effects giving uniform dyeability, suppressing damage of hair on dyeing, and imparting gloss, springness and combability to hairs.

Even after washing 20 times with shampoo, the evaluated values of the hair dyed by the hair coloring agent of Example 11 still higher in every evaluation items in comparison with the hair dyed by the hair coloring agent of Comparative example 16. From this result, it is clear that silylated peptide-silane compound copolymer composition blended in the hair coloring agent of Example 11 improve shampoo fastness of dyed hair.

### Example 12 and Comparative Example 18 and 19

First compositions of hair coloring agents having composition as shown in Table 21 were prepared, and evaluated about uniform dyeability, and gloss, springness and combability of the dyed hairs.

In Example 12, N-[2-hydroxy-3-(3'-methyldihydroxysilyl)propoxy]propyl hydrolyzed silk-dimethyldiethoxysilane-octyltriethoxysilane copolymer composition produced in Production Example 3 was used. In Comparative Example 18, in place of a silylated peptide-silane compound copolymer composition, octamethylcyclotetrasiloxane (SH244 (trade name), manufactured by Dow Corning Toray Silicone Co.,Ltd.) was used; in Comparative Example 19 (Control, blank), neither a silylated peptide-silane compound copolymer composition nor a silicone were used.

**Table 21**

| | Example | Comparative example | |
|---|---|---|---|
| | 12 | 18 | 19 |
| Silylated peptide-silane compound copolymer composition (70%) of Production Example 3 | 1.5 | 0 | 0 |
| Octamethylcyclo tetrasiloxane | 0 | 1.05 | 0 |
| p-phenylenediamine | 1.0 | 1.0 | 1.0 |
| Propyleneglycol | 10.0 | 10.0 | 10.0 |
| Ammonium tioglycolate (50%) | 0.5 | 0.5 | 0.5 |
| Polyoxyethylene(6)stearyl ether | 1.0 | 1.0 | 1.0 |
| Edetate disodium | 0.3 | 0.3 | 0.3 |
| Aqueous ammonia (28%) Aqueous ammonia (28%) | To adjust pH 10 | To adjust pH 10 | To adjust pH 10 |
| Sterilized ion-exchange water | Volume adjustable totally 100 part | Volume adjustable totally 100 part | Volume adjustable totally 100 part |

Second compositions of hair coloring agents having composition as shown in Table 21-2 were prepared.

**Table 21-2**

| | Example | Comparative example | |
|---|---|---|---|
| | 12 | 18 | 19 |
| Hydrogen peroxide (35%) | 6.0 | 6.0 | 6.0 |
| Sterilized ion-exchange water | 94.0 | 94.0 | 94.0 |

Three bundles of hair, each bundle being the length of 10 cm and the weight of 1 g, were prepared, and bleached according to the same manner as in Example 10. Then dyeing was carried out using the hair coloring agent of Example 12, Comparative example 18 or Comparative example 19.

Dyeing was carried out by mixing same amounts of the first composition and the second composition, coating 2 g of the mixture on each hair bundle, then leaving the bundle for 20 minutes, rinsing it with hot water, and washing it with 2% aqueous solution of sodium polyoxyethylene(3) laurylether sulfate. After dyeing was completed, the hair bundle was dried with a hair dryer, and the obtained hair bundle was evaluated about uniform dyeability, gloss, springness and combability by ten panelists according to the same rating as in Example 10.

Further, a bundle of dyed hair with the hair coloring agent of Example 12, Comparative example 18 or Comparative example 19 was washed with 2% aqueous solution of sodium polyoxyethylene(3) laurylether sulfate, rinsed with water and dried with a hair dryer. After repeating above procedure 20 times, the washed hair bundle was evaluated about depth of color after washing, gloss, springness and combability by ten panelists according to the same rating as in Example 10. The results indicate shampoo fastness and are shown in Table 22 in terms of averaged value of ten panelists.

**Table 22**

| | | Example | Comparative example |
|---|---|---|---|
| | | 12 | 18 |
| Dyed hair before washing: | | | |
| | Uniform dyeability | +2.5 | +1.4 |
| | Gloss | +2.4 | +1.4 |
| | Springness | +2.4 | +0.9 |
| | Combability | +2.5 | +1.1 |
| Dyed hair after washing: | | | |
| | Color Depth | +2.7 | +0.6 |
| | Gloss | +2.2 | +1.2 |
| | Springness | +2.6 | +0.4 |
| | Combability | +2.2 | +0.9 |

As shown in Table 22, the evaluated values of the hair dyed by the hair coloring agent of Example 12 blended with N-[2-hydroxy-3-(3'-methyldihydroxysilyl)propoxy]propyl hydrolyzed silk-dimethyldiethoxysilane-octyltriethoxysilane copolymer composition are rated plus(+), that is, better than the hair dyed by the hair coloring agent of Comparative example 19 (Control), and are higher in every evaluation item in comparison with the hair dyed by the hair coloring agent of Comparative example 18 blended with an amino modifiedsilicone.Therefore,thesilylated peptide-silane compound copolymer composition blended in the hair coloring agent of Example 12 clearly exerts excellent effects giving uniform dyeability, suppressing damage of hair on dyeing, and imparting gloss, springness and combability to hairs.

Even after washing 20 times with shampoo, the evaluated values of the hair dyed by the hair coloring agent of Example 12 still higher in every evaluation items in comparison with the hair dyed by the hair coloring agent of Comparative example 18. From this result, it is clear that silylated peptide-silane compound copolymer composition blended in the hair coloring agent of Example 12 improve shampoo fastness of dyed hair.

## Claims

1. A hair treatment agent, a shampoo or a hair coloring agent comprising a silylated peptide-silane compound copolymer composition which is obtainable by the following process and has a viscosity in the range from 500 to 2000 mPa· s in 70% of solid content concentration of said copolymer composition at 20 °C,
wherein said process comprises:
polycondensing one or more kind selected from silylated peptides represented by the general formula (I) with one or more kind selected from silane compound represented by the general formula (II) in an aqueous solution in a range of reaction molar ratio of said silylated peptide to said silane compound from 1:50 to 1:85 to form a polycondensed polymer, and then
adding by addition reaction a silane compound represented by the general formula (III) in an aqueous solution to said polycondensed polymer;
wherein said silylated peptide represented by the following general formula (I) is 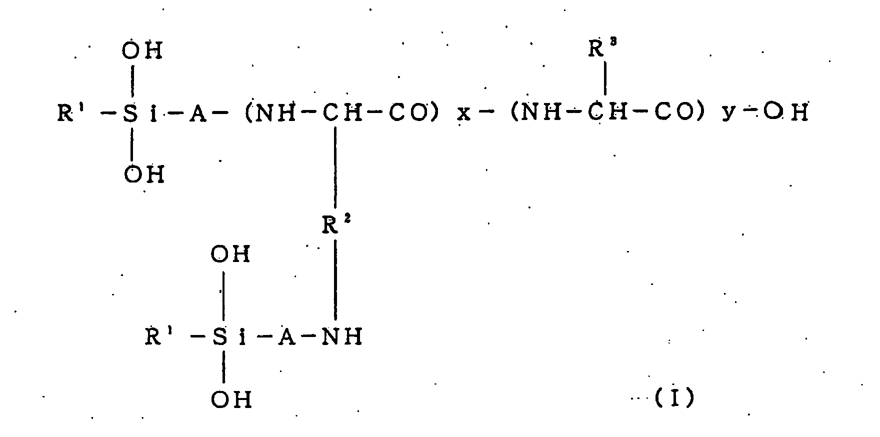 , in which R¹ represents a hydroxy group or an alkyl group having 1 to 3 carbon atoms, R² represents a residual group of a side chain obtained by removing the terminal end amino group of a basic amino acid having an amino group at the end of a side chain, R³ represents a side chain of an amino acid other than R², A is a connecting moiety and represents at least one group selected from the group consisting of -CH₂-, (CH₂)₃-, -(CH₂)₃OCH₂CH(OH)CH₂-, -(CH₂)₃S-, -(CH₂)₃NH- and -(CH₂)₃OCOCH₂CH₂-, and x is from 0 to 50, y is from 1 to 100 and x+y is from 1 to 100, wherein x and y represent only the number of amino acid units and do not represent the order of amino acid sequence; wherein said silane compound represented by the following general formula (II) is
R⁴mSi(OH)pY(4-p-m) (II),
in which m represents an integer from 0 to 2, p represents an integer from 2 to 4, m+p is not more than 4, R⁴ represents an organic group in which a carbon atom is directly connected to the silicon atom and the R⁴s of m may be the same or different, and the Ys of (4-p-m) represent an alkoxy group or hydrogen atom; and
wherein the silane compound represented by the following general formula (III) is
R⁵₃Si (OH) (III),
wherein the three R⁵s represent organic groups in which a carbon atom is directly connected to the silicon atom and the three R⁵s may be the same or different.

2. A hair coloring agent according to claim 1, wherein the silylated peptide-silane compound copolymer composition is comprised in an amount of from 0.05 to 10 weight % of the total amount of the agent.

## Patentansprüche

1. Haarbehandlungsmittel, Shampoo oder Haarfärbemittel, umfassend eine silyliertes Peptid-Silanverbindung-Copolymerzusammensetzung, welche durch das folgende Verfahren erhältlich ist und eine Viskosität im Bereich von 500 bis 20000 mPa·s bei 70% Feststoffgehaltskonzentration der Copolymerzusammensetzung bei 20°C aufweist,
wobei das Verfahren umfasst:
Polykondensation einer oder mehrerer Arten, ausgewählt aus silylierten Peptiden der allgemeinen Formel (I), mit einer oder mehreren Arten, ausgewählt aus Silanverbindungen der allgemeinen Formel (II), in einer wässrigen Lösung in einem Bereich des Reaktions-Molverhältnisses des silylierten Peptids zu der Silanverbindung von 1:50 bis 1:85, um ein polykondensiertes Polymer zu bilden, und anschließend
Zugabe durch Additionsreaktion einer Silanverbindung der allgemeinen Formel (III) in einer wässrigen Lösung zu dem polykondensierten Polymer;
wobei das silylierte Peptid die folgende allgemeine Formel (I) 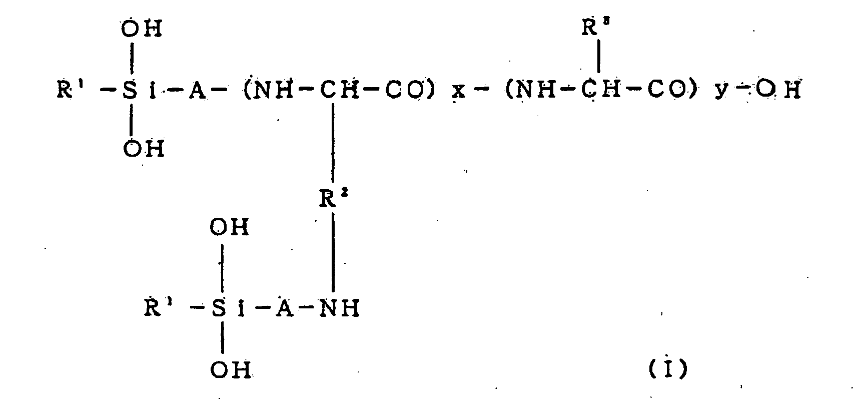 aufweist, wobei R¹ eine Hydroxygruppe oder einen Alkylrest mit 1 bis 3 Kohlenstoffatomen bedeutet, R² einen Rest einer Seitenkette bedeutet, der durch Entfernen der endständigen Aminogruppe einer basischen Aminosäure mit einer Aminogruppe am Ende einer Seitenkette erhalten wurde, R³ eine von R² verschiedene Seitenkette einer Aminosäure bedeutet, A eine Verbindungseinheit ist und mindestens einen Rest, ausgewählt aus -CH₂-, -(CH₂)₃-, -(CH₂)₃OCH₂CH(OH)CH₂-, -(CH₂)₃S-, -(CH₂)₃NH- und -(CH₂)₃OCOCH₂CH₂-, darstellt, und x 0 bis 50 beträgt, y 1 bis 100 beträgt und x+y 1 bis 100 beträgt, wobei x und y nur die Anzahl an Aminosäureeinheiten bedeuten und nicht die Reihenfolge der Aminosäuresequenz wiedergeben;
wobei die Silanverbindung die folgende allgemeine Formel (II)
R⁴mSi(OH)pY(4-p-m) (II),
aufweist, wobei m eine ganze Zahl von 0 bis 2 bedeutet, p eine ganze Zahl von 2 bis 4 bedeutet, m+p nicht mehr als 4 beträgt, R⁴ einen organischen Rest bedeutet, in welchem ein Kohlenstoffatom direkt an das Siliciumatom gebunden ist, und die zu m gehörigen Reste R⁴ gleich oder verschieden sein können, und die zu (4-p-m) gehörigen Reste Y einen Alkoxyrest oder ein Wasserstoffatom bedeuten; und
wobei die Silanverbindung die folgende allgemeine Formel (III)
R⁵₃Si(OH) (III)
aufweist, wobei die drei Reste R⁵ organische Reste darstellen, in welchen ein Kohlenstoffatom direkt an das Siliciumatom gebunden ist, und die drei Reste R⁵ gleich oder verschieden sein können.

2. Haarfärbemittel nach Anspruch 1, wobei die silyliertes Peptid-Silanverbindung-Copolymerzusammensetzung in einer Menge von 0,05 bis 10 Gew.-% der Gesamtmenge des Mittels enthalten ist.

## Revendications

1. Agent de traitement capillaire, shampooing ou agent de coloration capillaire comprenant une composition de copolymère de peptide silylé - composé silane qui peut être obtenue par le procédé suivant et a une viscosité dans la plage de 500 à 20000 mPa.s dans une concentration ayant une teneur en solide de 70 % de ladite composition de copolymère à 20 °C,
où ledit procédé comprend les étapes de :
polycondenser un ou plusieurs composés choisis parmi les peptides silylés représentés par la formule générale (I) avec un ou plusieurs composés choisis parmi les composés silanes représentés par la formule générale (II) dans une solution aqueuse selon un rapport molaire réactionnel dudit peptide silylé audit composé silane compris dans la plage de 1/50 à 1/85 pour former un polymère polycondensé, puis
ajouter par réaction d'addition un composé silane représenté par la formule générale (III) dans une solution aqueuse audit polymère polycondensé ;
où ledit peptide silylé représenté par la formule générale suivante (I) est 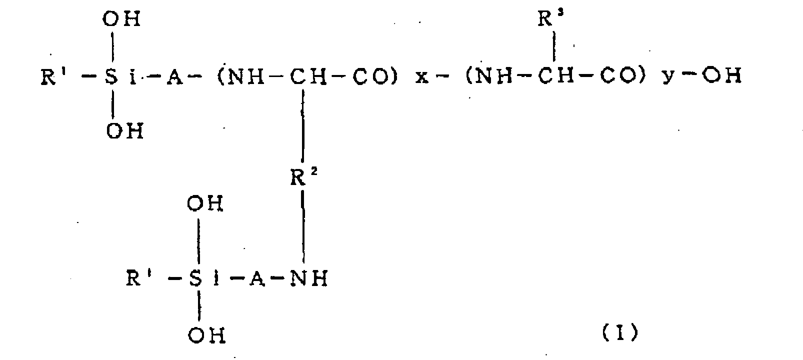
dans laquelle R¹ représente un groupe hydroxy ou un groupe alkyle ayant de 1 à 3 atomes de carbone, R² représente un groupe résiduaire d'une chaîne latérale obtenue par l'élimination du groupe amino terminal d'un acide aminé basique ayant un groupe amino en position terminale d'une chaîne latérale, R³ représente une chaîne latérale d'un acide aminé autre que R², A est un fragment liant et représente au moins un groupe choisi dans le groupe constitué par -CH₂-, -(CH₂)₃-, -(CH₂)₃OCH₂CH(OH)CH₂-, -(CH₂)₃S-, -(CH₂)₃NH- et -(CH₂)₃OCOCH₂CH₂-, et x vaut de 0 à 50, y vaut de 1 à 100 et x+y vaut de 1 à 100, où x et y représentent seulement le nombre de motifs d'acide aminé et ne représentent pas l'ordre de la séquence d'acide aminé ;
où ledit composé silane représenté par la formule générale (II) suivante est
R⁴mSi (OH)pY(4-p-m) (II)
dans laquelle m représente un entier de 0 à 2, p représente un entier de 2 à 4, m+p n'est pas supérieur à 4, R⁴ représente un groupe organique dans lequel un atome de carbone est directement lié à l'atome de silicium et les R⁴ de m peuvent être identiques ou différents les uns des autres, et les Y de (4-p-m) représentent un groupe alcoxy ou un atome d'hydrogène ; et
où le composé silane représenté par la formule générale (III) suivante est
R⁵₃Si(OH) (III)
où les trois R⁵ représentent des groupes organiques dans lesquels un atome de carbone est directement lié à l'atome de silicium et les trois R⁵ peuvent être identiques ou différents les uns des autres.

2. Agent de coloration capillaire selon la revendication 1, dans lequel la composition de copolymère de peptide silylé - composé silane est comprise en une quantité de 0,05 à 10 % en poids de la quantité totale de l'agent.
